# EUROPEAN PATENT APPLICATION

(11) **EP 3 560 956 A2**
(43) Date of publication of application: **30.10.2019**
(21) Application number: 19176173.3
(22) Date of filing: 13.04.2017
(51) Int. Cl.: C07K 16/24, A61P 17/06, A61K 39/395

(54) **METHODS OF TREATING INFLAMMATORY DISEASES**

(30) Priority: 15.04.2016 US 201662323075 P; 16.05.2016 US 201662336815 P; 20.09.2016 US 201662396855 P; 27.09.2016 US 201662400116 P; 27.02.2017 US 201762463959 P
(62) Divisional of application: 17719786.0
(71) Applicant: Boehringer Ingelheim International GmbH, 55216 Ingelheim am Rhein (DE)
(72) Inventor: Baum, Patrick, 55216 Ingelheim am Rhein (DE); Flack, Mary Ruth, Ridgefield, Connecticut 06877-0368 (US); Galler, Annette Bettina, 55216 Ingelheim am Rhein (DE); Lalovic, Bojan, Ridgefield, Connecticut 06877-0368 (US); Maino, Kaori, 55216 Ingelheim am Rhein (DE); Padula, Steven John, Ridgefield, Connecticut 06877-0368 (US); Scholl, Paul, Ridgefield, Connecticut 06877-0368 (US); Sha, Koji, 55216 Ingelheim am Rhein (DE); Visvanathan, Sudha, Ridgefield, Connecticut 06877-0368 (US)
(74) Representative: Hoffmann Eitle

(57) **Abstract**

This invention generally relates to methods for the treatment of IL-23 related diseases, in particular inflammatory diseases, such as psoriasis or psoriatic arthritis, utilizing anti-IL-23 A antibodies.

SEQUENCE LISTING

<110> BOEHRINGER INGELHEIM INTERNATIONAL GMBH

<120> METHODS OF TREATING INFLAMMATORY DISEASES

<130> 114758-0134

<140>
<141>

<150> 62/463,959
<151> 2017-02-27

<150> 62/400,116
<151> 2016-09-27

<150> 62/396,855
<151> 2016-09-20

<150> 62/336,815
<151> 2016-05-16

<150> 62/323,075
<151> 2016-04-15

<160> 22

<170> PatentIn version 3.5

<210> 1
<211> 11
<212> PRT
<213> Mus sp.

<400> 1
Lys Ala Ser Arg Asp Val Ala Ile Ala Val Ala
1               5                   10

<210> 2
<211> 7
<212> PRT
<213> Mus sp.

<400> 2
Trp Ala Ser Thr Arg His Thr
1               5

<210> 3
<211> 9
<212> PRT
<213> Mus sp.

<400> 3
His Gln Tyr Ser Ser Tyr Pro Phe Thr
1               5

<210> 4
<211> 10
<212> PRT

<213> Mus sp.

<400> 4
Gly Asn Thr Phe Thr Asp Gln Thr Ile His
1               5                   10


<210> 5
<211> 17
<212> PRT
<213> Mus sp.

<400> 5
Tyr Ile Tyr Pro Arg Asp Asp Ser Pro Lys Tyr Asn Glu Asn Phe Lys
1               5                   10                  15


Gly




<210> 6
<211> 11
<212> PRT
<213> Mus sp.

<400> 6
Pro Asp Arg Ser Gly Tyr Ala Trp Phe Ile Tyr
1               5                   10


<210> 7
<211> 10
<212> PRT
<213> Artificial Sequence

<220>
<223> Description of Artificial Sequence: Humanized
      sequence

<400> 7
Gly Tyr Thr Phe Thr Asp Gln Thr Ile His
1               5                   10


<210> 8
<211> 10
<212> PRT
<213> Artificial Sequence

<220>
<223> Description of Artificial Sequence: Humanized
      sequence

<400> 8
Gly Phe Thr Phe Thr Asp Gln Thr Ile His
1               5                   10


<210> 9
<211> 10
<212> PRT
<213> Artificial Sequence

<220>
<223> Description of Artificial Sequence: Humanized
      sequence

<400> 9
Gly Gly Thr Phe Thr Asp Gln Thr Ile His
1               5                   10


<210> 10
<211> 107
<212> PRT
<213> Artificial Sequence

<220>
<223> Description of Artificial Sequence: Humanized
      sequence

<400> 10
Asp Ile Gln Met Thr Gln Ser Pro Ser Ser Leu Ser Ala Ser Val Gly
1               5                   10                  15


Asp Arg Val Thr Ile Thr Cys Lys Ala Ser Arg Asp Val Ala Ile Ala
            20                  25                  30


Val Ala Trp Tyr Gln Gln Lys Pro Gly Lys Val Pro Lys Leu Leu Leu
        35                  40                  45


Phe Trp Ala Ser Thr Arg His Thr Gly Val Pro Asp Arg Phe Ser Gly
    50                  55                  60


Ser Gly Ser Gly Thr Asp Phe Thr Leu Thr Ile Ser Ser Leu Gln Pro
65                  70                  75                  80


Glu Asp Leu Ala Asp Tyr Tyr Cys His Gln Tyr Ser Ser Tyr Pro Phe
                85                  90                  95


Thr Phe Gly Gln Gly Thr Lys Leu Glu Ile Lys
            100                 105


<210> 11
<211> 107
<212> PRT
<213> Artificial Sequence

<220>
<223> Description of Artificial Sequence: Humanized
      sequence

<400> 11
Asp Ile Gln Met Thr Gln Ser Pro Ser Ser Leu Ser Ala Ser Val Gly
1               5                   10                  15


Asp Arg Val Thr Ile Thr Cys Lys Ala Ser Arg Asp Val Ala Ile Ala

20                  25                  30

Val Ala Trp Tyr Gln Gln Lys Pro Gly Lys Val Pro Lys Leu Leu Ile
        35                  40                  45

Tyr Trp Ala Ser Thr Arg His Thr Gly Val Pro Ser Arg Phe Ser Gly
    50                  55                  60

Ser Gly Ser Arg Thr Asp Phe Thr Leu Thr Ile Ser Ser Leu Gln Pro
65                  70                  75                  80

Glu Asp Val Ala Asp Tyr Phe Cys His Gln Tyr Ser Ser Tyr Pro Phe
                85                  90                  95

Thr Phe Gly Ser Gly Thr Lys Leu Glu Ile Lys
            100                 105

<210> 12
<211> 107
<212> PRT
<213> Artificial Sequence

<220>
<223> Description of Artificial Sequence: Humanized
      sequence

<400> 12
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Tyr Trp Ala Ser Thr Arg His Thr Gly Val Pro Ser Arg Phe Ser Gly
    50                  55                  60

Ser Gly Ser Arg Thr Asp Phe Thr Leu Thr Ile Ser Ser Leu Gln Pro
65                  70                  75                  80

Glu Asp Val Ala Thr Tyr Tyr Cys His Gln Tyr Ser Ser Tyr Pro Phe
                85                  90                  95

Thr Phe Gly Ser Gly Thr Lys Leu Glu Ile Lys
            100                 105

<210> 13
<211> 107

<212> PRT
<213> Artificial Sequence

<220>
<223> Description of Artificial Sequence: Humanized
      sequence

<400> 13
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Phe Trp Ala Ser Thr Arg His Thr Gly Val Pro Asp Arg Phe Ser Gly
    50                  55                  60

Ser Gly Ser Arg Thr Asp Phe Thr Leu Thr Ile Ser Ser Leu Gln Pro
65                  70                  75                  80

Glu Asp Leu Ala Asp Tyr Tyr Cys His Gln Tyr Ser Ser Tyr Pro Phe
                85                  90                  95

Thr Phe Gly Ser Gly Thr Lys Leu Glu Ile Lys
            100                 105

<210> 14
<211> 120
<212> PRT
<213> Artificial Sequence

<220>
<223> Description of Artificial Sequence: Humanized
      sequence

<400> 14
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Gln
            20                  25                  30

Thr Ile His Trp Met Arg Gln Ala Pro Gly Gln Gly Leu Glu Trp Ile
        35                  40                  45

Gly Tyr Ile Tyr Pro Arg Asp Asp Ser Pro Lys Tyr Asn Glu Asn Phe
    50                  55                  60

Lys Gly Lys Val Thr Ile Thr Ala Asp Lys Ser Thr Ser Thr Ala Tyr

65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser
        115                 120

<210> 15
<211> 120
<212> PRT
<213> Artificial Sequence

<220>
<223> Description of Artificial Sequence: Humanized
      sequence

<400> 15
Gln Val Gln Leu Val Gln Ser Gly Ala Glu Val Lys Lys Pro Gly Ser
1               5                   10                  15

Ser Val Lys Val Ser Cys Lys Ala Ser Gly Phe Thr Phe Thr Asp Gln
            20                  25                  30

Thr Ile His Trp Val Arg Gln Ala Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Lys Val Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser
        115                 120

<210> 16
<211> 120
<212> PRT
<213> Artificial Sequence

<220>

<223> Description of Artificial Sequence: Humanized
      sequence

<400> 16
Gln Val Gln Leu Val Gln Ser Gly Ala Glu Val Lys Lys Pro Gly Ser
1               5                   10                  15

Ser Val Lys Thr Ser Cys Lys Ala Ser Gly Gly Thr Phe Thr Asp Gln
            20                  25                  30

Thr Ile His Trp Val Arg Gln Arg Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser
        115                 120


<210> 17
<211> 120
<212> PRT
<213> Artificial Sequence

<220>
<223> Description of Artificial Sequence: Humanized
      sequence

<400> 17
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Thr Asp Gln
            20                  25                  30

Thr Ile His Trp Val Arg Gln Ala Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Arg Val Thr Leu Thr Ala Asp Lys Ser Thr Ser Thr Ala Tyr

65                  70                  75                  80

Met Glu Leu Ser Ser Leu Arg Ser Glu Asp Thr Ala Val Tyr Phe Cys
                85                  90                  95

Ala Arg Pro Asp Arg Ser Gly Tyr Ala Trp Phe Ile Tyr Trp Gly Gln
            100                 105                 110

Gly Thr Leu Val Thr Val Ser Ser
        115                 120

<210> 18
<211> 214
<212> PRT
<213> Artificial Sequence

<220>
<223> Description of Artificial Sequence: Humanized
      sequence

<400> 18
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Ile
        35                  40                  45

Tyr Trp Ala Ser Thr Arg His Thr Gly Val Pro Ser Arg Phe Ser Gly
    50                  55                  60

Ser Gly Ser Arg Thr Asp Phe Thr Leu Thr Ile Ser Ser Leu Gln Pro
65                  70                  75                  80

Glu Asp Val Ala Asp Tyr Phe Cys His Gln Tyr Ser Ser Tyr Pro Phe
                85                  90                  95

Thr Phe Gly Ser Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 19
<211> 449
<212> PRT
<213> Artificial Sequence

<220>
<223> Description of Artificial Sequence: Humanized
      sequence

<400> 19
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Gln
            20                  25                  30

Thr Ile His Trp Met Arg Gln Ala Pro Gly Gln Gly Leu Glu Trp Ile
        35                  40                  45

Gly Tyr Ile Tyr Pro Arg Asp Asp Ser Pro Lys Tyr Asn Glu Asn Phe
    50                  55                  60

Lys Gly Lys Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210> 20
<211> 449
<212> PRT
<213> Artificial Sequence

<220>
<223> Description of Artificial Sequence: Humanized
      sequence

<400> 20
Gln Val Gln Leu Val Gln Ser Gly Ala Glu Val Lys Lys Pro Gly Ser
1               5                   10                  15

Ser Val Lys Val Ser Cys Lys Ala Ser Gly Phe Thr Phe Thr Asp Gln
            20                  25                  30

Thr Ile His Trp Val Arg Gln Ala Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Lys Val Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210> 21
<211> 214
<212> PRT
<213> Artificial Sequence

<220>
<223> Description of Artificial Sequence: Humanized
sequence

<400> 21
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Phe Trp Ala Ser Thr Arg His Thr Gly Val Pro Asp Arg Phe Ser Gly
    50                  55                  60

Ser Gly Ser Gly Thr Asp Phe Thr Leu Thr Ile Ser Ser Leu Gln Pro
65                  70                  75                  80

Glu Asp Leu Ala Asp Tyr Tyr Cys His Gln Tyr Ser Ser Tyr Pro Phe
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 22
<211> 189
<212> PRT
<213> Homo sapiens

<400> 22
Met Leu Gly Ser Arg Ala Val Met Leu Leu Leu Leu Leu Pro Trp Thr
1               5                   10                  15

Ala Gln Gly Arg Ala Val Pro Gly Gly Ser Ser Pro Ala Trp Thr Gln
            20                  25                  30

Cys Gln Gln Leu Ser Gln Lys Leu Cys Thr Leu Ala Trp Ser Ala His
        35                  40                  45

Pro Leu Val Gly His Met Asp Leu Arg Glu Glu Gly Asp Glu Glu Thr
    50                  55                  60

Thr Asn Asp Val Pro His Ile Gln Cys Gly Asp Gly Cys Asp Pro Gln
65                  70                  75                  80

Gly Leu Arg Asp Asn Ser Gln Phe Cys Leu Gln Arg Ile His Gln Gly
                85                  90                  95

Leu Ile Phe Tyr Glu Lys Leu Leu Gly Ser Asp Ile Phe Thr Gly Glu
            100                 105                 110

Pro Ser Leu Leu Pro Asp Ser Pro Val Gly Gln Leu His Ala Ser Leu
        115                 120                 125

Leu Gly Leu Ser Gln Leu Leu Gln Pro Glu Gly His His Trp Glu Thr
    130                 135                 140

Gln Gln Ile Pro Ser Leu Ser Pro Ser Gln Pro Trp Gln Arg Leu Leu
145                 150                 155                 160

Leu Arg Phe Lys Ile Leu Arg Ser Leu Gln Ala Phe Val Ala Val Ala
                165                 170                 175

Ala Arg Val Phe Ala His Gly Ala Ala Thr Leu Ser Pro
            180                 185

## Description

### CROSS-REFERENCE TO RELATED APPLICATIONS

This application claims priority from U.S. Provisional Application Nos. 62/323,075, filed April 15, 2016, 62/336,815, filed May 16, 2016, 62/396,855, filed September 20, 2016, 62/400,116, filed September 27, 2016, and 62/463,959, filed February 27, 2017, the entire contents of which are incorporated herein.

The instant application contains a Sequence Listing which has been submitted in ASCII format via EFS-WEB and is hereby incorporated by reference in its entirety. Said ASCII copy, created on April 7, 2017, is named sequence.txt and is 24 KB.

### TECHNICAL FIELD OF THE INVENTION

This invention generally relates to method of treating inflammatory diseases, for example psoriasis, psoriatic arthritis or axial (spinal) spondyloarthritis (ax-SpA), including ankylosing spondylitis (AS, also called radiographic ax-SpA) and non-radiographic ax-SpA, utilizing anti-IL-23A antibodies.

### BACKGROUND OF THE INVENTION

Psoriasis is a chronic, immune-mediated, inflammatory skin disease, with a global incidence of approximately 2%, associated with significant morbidity and can have a substantial psychosocial impact on quality of life and well-being of patients. Plaque psoriasis is the most common form and affects approximately 80-90% of patients, manifesting as raised plaques on the skin; the disease usually begins in late adolescence and early adulthood and may persist through adult life. The extent of the affected body surface area (BSA) and the degree of skin manifestations, including erythema, induration, and scaling, defines the severity of psoriasis with approximately 20-30% of patients having moderate-to-severe disease. Approximately one third of patients with psoriasis have associated joint inflammation (psoriatic arthritis (PsA)) resulting in pain and disability. Axial (spinal) spondyloarthritis (ax-SpA), including ankylosing Spondylitis (AS, also called radiographic axial spondyloarthritis (ax-SpA)) and non-radiographic axial spondyloarthritis (ax-SpA), are an inflammatory disease involving primarily the axial skeleton and sacroiliac joints.

Psoriasis, psoriatic arthritis and axial (spinal) spondyloarthritis (ax-SpA), which includes ankylosing spondylitis and non-radiographic ax-SpA, are multifactorial autoimmune diseases whose exact aetiology is unknown. Multiple genome-wide association studies have linked variants in the genes for the IL-23 receptor to psoriasis susceptibility. Human IL-23 is primarily produced by antigen presenting cells and induces differentiation of T helper 17 (Th17) cells. This results in the production of IL-17 and IL-22, which may mediate the development of the epidermal hyperplasia and tissue inflammation observed in psoriasis.

There is a need for treatment options for inflammatory diseases, in particular psoriasis, psoriatic arthritis, axial (spinal) spondyloarthritis (ax-SpA), including ankylosing spondylitis and non-radiographic ax-SpA, that lead to favorable outcomes for patients, for example in terms of efficacy, safety and/or tolerability of the treatment.

### SUMMARY OF THE INVENTION

The present invention addresses the above needs and provides methods for treating inflammatory diseases, in particular methods comprising administering an anti-IL-23A antibody to a patient in certain amounts and/or at certain intervals. In one aspect, a method of the present invention is for the treatment of psoriasis or psoriatic arthritis. In one aspect, a method of the present invention is for the treatment of axial (spinal) spondyloarthritis (ax-SpA), including ankylosing spondylitis (AS) and non-radiographic ax-SpA. Accordingly, in one aspect, a method of the present invention is for the treatment of ankylosing spondylitis (AS) and in one aspect, a method of the present invention is for the treatment of non-radiographic ax-SpA.

The methods of the present invention provide the advantage of enabling patients to experience clinical improvement while receiving fewer administrations of the anti-IL-23A antibody.

In one embodiment, the present invention provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of the anti-IL-23A antibody to the patient;
b) administering a first maintenance dose of the anti-IL-23A antibody to the patient 4 to 24 weeks, for example 4 to 16 weeks, for example 4 to 12 weeks, after said initial dose is administered; and
c) administering at least one additional maintenance dose to the patient 4 to 24 weeks, for example 4 to 16 weeks, for example 4 to 12 weeks, after said first maintenance dose is administered.

In one embodiment, the present invention provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of the anti-IL-23A antibody to the patient;
b) administering a first maintenance dose of the anti-IL-23A antibody to the patient 8 to 24 weeks, for example 8 to 16 weeks, for example 8 to 12 weeks, for example 6 to 24 weeks, for example 6 to 16 weeks, for example 6 to 12 weeks, after said initial dose is administered; and
c) administering at least one additional maintenance dose to the patient 8 to 24 weeks, for example 8 to 16 weeks, for example 8 to 12 weeks, for example 6 to 24 weeks, for example 6 to 16 weeks, for example 6 to 12 weeks, after said first maintenance dose is administered.

In one embodiment, the first maintenance dose is administered to the patient 4, 6, 8, 12, 16, 20 or 24 weeks after the initial dose is administered.

In one embodiment, the at least one additional maintenance dose is administered to the patient 4, 6, 8, 12, 16, 20 or 24 weeks after the first maintenance dose is administered.

In one embodiment, the first maintenance dose is administered to the patient 4, 6, 8, 12, 16, 20 or 24 weeks after the initial dose is administered and the at least one additional maintenance dose is administered to the patient 4, 6, 8, 12, 16, 20 or 24 weeks after the first maintenance dose is administered.

In additional embodiments:
the first maintenance dose is administered to the patient 4 weeks after the initial dose is administered and the at least one additional maintenance dose is administered to the patient 4 weeks after the first maintenance dose is administered; or
the first maintenance dose is administered to the patient 6 weeks after the initial dose is administered and the at least one additional maintenance dose is administered to the patient 6 weeks after the first maintenance dose is administered; or
the first maintenance dose is administered to the patient 8 weeks after the initial dose is administered and the at least one additional maintenance dose is administered to the patient 8 weeks after the first maintenance dose is administered; or
the first maintenance dose is administered to the patient 12 weeks after the initial dose is administered and the at least one additional maintenance dose is administered to the patient 12 weeks after the first maintenance dose is administered; or
the first maintenance dose is administered to the patient 16 weeks after the initial dose is administered and the at least one additional maintenance dose is administered to the patient 16 weeks after the first maintenance dose is administered; or
the first maintenance dose is administered to the patient 20 weeks after the initial dose is administered and the at least one additional maintenance dose is administered to the patient 20 weeks after the first maintenance dose is administered; or
the first maintenance dose is administered to the patient 24 weeks after the initial dose is administered and the at least one additional maintenance dose is administered to the patient 24 weeks after the first maintenance dose is administered.

In one embodiment, in any one of the methods above, the initial dose comprises 15 to 300 mg, for example 15 to 250 mg, for example 90 to 180 mg, of the anti- IL-23A antibody.

In one embodiment, in any one of the methods above, the first maintenance dose and the at least one additional maintenance dose comprise 15 to 300 mg, for example 15 to 250 mg, for example 90 to 180 mg, of the anti- IL-23A antibody.

In one embodiment, in any one of the methods above, the initial dose comprises 70 to 90 mg, 80 to 100 mg, 90 to 110 mg, 100 to 120 mg, 110 to 130 mg, 120 to 140 mg, 130 to 150 mg, 140 to 160 mg, 150 to 170 mg, 160 to 180 mg, 170 to 190 mg, 180 to 200 mg, 190 to 210 mg, 200 to 220 mg, 210 to 230 mg, 220 to 240 mg, 230 to 250 mg, 240 to 260 mg, 250 to 270 mg, 260 to 280 mg, 270 to 290 mg or 280 to 300 mg of the anti- IL-23A antibody.

In one embodiment, in any one of the methods above, the first maintenance dose and the at least one additional maintenance dose comprise 70 to 90 mg, 80 to 100 mg, 90 to 110 mg, 100 to 120 mg, 110 to 130 mg, 120 to 140 mg, 130 to 150 mg, 140 to 160 mg, 150 to 170 mg, 160 to 180 mg, 170 to 190 mg, 180 to 200 mg, 190 to 210 mg, 200 to 220 mg, 210 to 230 mg, 220 to 240 mg, 230 to 250 mg, 240 to 260 mg, 250 to 270 mg, 260 to 280 mg, 270 to 290 mg or 280 to 300 mg of the anti- IL-23A antibody.

In one embodiment, in any one of the methods above, the initial dose comprises 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of the anti- IL-23A antibody. In one embodiment, in any one of the methods above, the initial dose comprises 75 mg of the anti- IL-23A antibody.

In one embodiment, in any one of the methods above, the first maintenance dose and the at least one additional maintenance dose comprise 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of the anti- IL-23A antibody. In one embodiment, in any one of the methods above, the first maintenance dose and the at least one additional maintenance dose comprise 75 mg of the anti- IL-23A antibody.

In one embodiment, in any one of the methods above, the initial dose, the first maintenance dose and the at least one additional maintenance dose comprise 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of the anti- IL-23A antibody. In one embodiment, in any one of the methods above, the initial dose, the first maintenance dose and the at least one additional maintenance dose comprise 75 mg of the anti- IL-23A antibody.

In one embodiment, the present invention provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of the anti-IL-23A antibody to the patient;
b) administering a first maintenance dose of the anti-IL-23A antibody to the patient 12 weeks after said initial dose is administered; and
c) administering at least one additional maintenance dose to the patient 12 weeks after said first maintenance dose is administered.

In one embodiment, in said method, the initial dose and the maintenance dose comprise 150 mg of said anti-IL-23A antibody. In one embodiment, in said method, the initial dose and the maintenance dose comprise 75 mg of said anti-IL-23A antibody.

In one embodiment, the present invention provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of the anti-IL-23A antibody to the patient;
b) administering a first maintenance dose of the anti-IL-23A antibody to the patient 8 weeks after said initial dose is administered; and
c) administering at least one additional maintenance dose to the patient 8 weeks after said first maintenance dose is administered.

In one embodiment, in said method, the initial dose and the maintenance dose comprise 90 mg of said anti-IL-23A antibody. In one embodiment, in said method, the initial dose and the maintenance dose comprise 150 mg of said anti-IL-23A antibody.

In one embodiment, the present invention provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of the anti-IL-23A antibody to the patient;
b) administering a first maintenance dose of the anti-IL-23A antibody to the patient 16 weeks after said initial dose is administered; and
c) administering at least one additional maintenance dose to the patient 16 weeks after said first maintenance dose is administered.

In one embodiment, in said method, the initial dose and the maintenance dose comprise 150 mg of said anti-IL-23A antibody.

In one embodiment, the present invention provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of the anti-IL-23A antibody to the patient;
b) administering a first maintenance dose of the anti-IL-23A antibody to the patient 6 weeks after said initial dose is administered; and
c) administering at least one additional maintenance dose to the patient 6 weeks after said first maintenance dose is administered.

In one embodiment, in said method, the initial dose and the maintenance dose comprise 150 mg of said anti-IL-23A antibody.

In one embodiment, the present invention provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of the anti-IL-23A antibody to the patient;
b) administering a first maintenance dose of the anti-IL-23A antibody to the patient 4 weeks after said initial dose is administered; and
c) administering at least one additional maintenance dose to the patient 4 weeks after said first maintenance dose is administered.

In one embodiment, in said method, the initial dose and the maintenance dose comprise 150 mg of said anti-IL-23A antibody.

In one embodiment, the present invention further provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of said anti-IL-23A antibody to the patient;
b) administering a loading dose of said anti-IL-23A antibody to the patient 1 to 6 weeks after said initial dose is administered;
c) administering a first maintenance dose of said anti-IL-23A antibody to the patient 8 to 24 weeks, for example 8 to 16 weeks, for example 8 to 12 weeks, for example 6 to 24 weeks, for example 6 to 16 weeks, for example 6 to 12 weeks, after said loading dose is administered; and
d) administering at least one additional maintenance dose to the patient 8 to 24 weeks, for example 8 to 16 weeks, for example 8 to 12 weeks, for example 6 to 24 weeks, for example 6 to 16 weeks, for example 6 to 12 weeks, after said first maintenance dose is administered.

In one embodiment, the loading dose is administered to the patient 1, 2, 3, 4, 5 or 6 weeks the said initial dose is administered.

In one embodiment, the first maintenance dose is administered to the patient 6, 8, 12, 16, 20 or 24 weeks after the loading dose is administered.

In one embodiment, the at least one additional maintenance dose is administered to the patient 6, 8, 12, 16, 20 or 24 weeks after the first maintenance dose is administered.

In one embodiment, the first maintenance dose is administered to the patient 6, 8, 12, 16, 20 or 24 weeks after the initial dose is administered and the at least one additional maintenance dose is administered to the patient 6, 8, 12, 16, 20 or 24 weeks after the first maintenance dose is administered.

In one embodiment, the first maintenance dose is administered to the patient 6, 8, 12, 16, 20 or 24 weeks after the loading dose is administered and the at least one additional maintenance dose is administered to the patient 6, 8, 12, 16, 20 or 24 weeks after the first maintenance dose is administered.

In additional embodiments:
the first maintenance dose is administered to the patient 6 weeks after the initial dose is administered and the at least one additional maintenance dose is administered to the patient 6 weeks after the first maintenance dose is administered; or
the first maintenance dose is administered to the patient 8 weeks after the initial dose is administered and the at least one additional maintenance dose is administered to the patient 8 weeks after the first maintenance dose is administered; or
the first maintenance dose is administered to the patient 12 weeks after the initial dose is administered and the at least one additional maintenance dose is administered to the patient 12 weeks after the first maintenance dose is administered; or
the first maintenance dose is administered to the patient 16 weeks after the initial dose is administered and the at least one additional maintenance dose is administered to the patient 16 weeks after the first maintenance dose is administered; or
the first maintenance dose is administered to the patient 20 weeks after the initial dose is administered and the at least one additional maintenance dose is administered to the patient 20 weeks after the first maintenance dose is administered; or
the first maintenance dose is administered to the patient 24 weeks after the initial dose is administered and the at least one additional maintenance dose is administered to the patient 24 weeks after the first maintenance dose is administered.

In additional embodiments:
the first maintenance dose is administered to the patient 6 weeks after the loading dose is administered and the at least one additional maintenance dose is administered to the patient 6 weeks after the first maintenance dose is administered; or
the first maintenance dose is administered to the patient 8 weeks after the loading dose is administered and the at least one additional maintenance dose is administered to the patient 8 weeks after the first maintenance dose is administered; or
the first maintenance dose is administered to the patient 12 weeks after the loading dose is administered and the at least one additional maintenance dose is administered to the patient 12 weeks after the first maintenance dose is administered: or
the first maintenance dose is administered to the patient 16 weeks after the loading dose is administered and the at least one additional maintenance dose is administered to the patient 16 weeks after the first maintenance dose is administered; or
the first maintenance dose is administered to the patient 20 weeks after the loading dose is administered and the at least one additional maintenance dose is administered to the patient 20 weeks after the first maintenance dose is administered; or
the first maintenance dose is administered to the patient 24 weeks after the loading dose is administered and the at least one additional maintenance dose is administered to the patient 24 weeks after the first maintenance dose is administered.

In one embodiment, in any one of the methods above, the initial dose comprises 15 to 300 mg, for example 15 to 250 mg, for example 90 to 180 mg, of the anti- IL-23A antibody.

In one embodiment, in any one of the methods above, the loading dose comprises 15 to 300 mg, for example 15 to 250 mg, for example 90 to 180 mg, of the anti- IL-23A antibody.

In one embodiment, in any one of the methods above, the first maintenance dose and the at least one additional maintenance dose comprise 15 to 300 mg, for example 15 to 250 mg, for example 90 to 180 mg, of the anti- IL-23A antibody.

In one embodiment, in any one of the methods above, the initial dose comprises 70 to 90 mg, 80 to 100 mg, 90 to 110 mg, 100 to 120 mg, 110 to 130 mg, 120 to 140 mg, 130 to 150 mg, 140 to 160 mg, 150 to 170 mg, 160 to 180 mg, 170 to 190 mg, 180 to 200 mg, 190 to 210 mg, 200 to 220 mg, 210 to 230 mg, 220 to 240 mg, 230 to 250 mg, 240 to 260 mg, 250 to 270 mg, 260 to 280 mg, 270 to 290 mg or 280 to 300 mg of the anti- IL-23A antibody.

In one embodiment, in any one of the methods above, the loading dose comprises 70 to 90 mg, 80 to 100 mg, 90 to 110 mg, 100 to 120 mg, 110 to 130 mg, 120 to 140 mg, 130 to 150 mg, 140 to 160 mg, 150 to 170 mg, 160 to 180 mg, 170 to 190 mg, 180 to 200 mg, 190 to 210 mg, 200 to 220 mg, 210 to 230 mg, 220 to 240 mg, 230 to 250 mg, 240 to 260 mg, 250 to 270 mg, 260 to 280 mg, 270 to 290 mg or 280 to 300 mg of the anti- IL-23A antibody.

In one embodiment, in any one of the methods above, the first maintenance dose and the at least one additional maintenance dose comprise 70 to 90 mg, 80 to 100 mg, 90 to 110 mg, 100 to 120 mg, 110 to 130 mg, 120 to 140 mg, 130 to 150 mg, 140 to 160 mg, 150 to 170 mg, 160 to 180 mg, 170 to 190 mg, 180 to 200 mg, 190 to 210 mg, 200 to 220 mg, 210 to 230 mg, 220 to 240 mg, 230 to 250 mg, 240 to 260 mg, 250 to 270 mg, 260 to 280 mg, 270 to 290 mg or 280 to 300 mg of the anti- IL-23A antibody.

In one embodiment, in any one of the methods above, the initial dose comprises 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of the anti-IL-23A antibody. In one embodiment, in any one of the methods above, the initial dose comprises 75 mg of the anti-IL-23A antibody.

In one embodiment, in any one of the methods above, the first maintenance dose and the at least one additional maintenance dose comprise 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of the anti-IL-23A antibody. In one embodiment, in any one of the methods above, the first maintenance dose and the at least one additional maintenance dose comprise 75 mg of the anti-IL-23A antibody.

In one embodiment, in any one of the methods above, the initial dose and the loading dose comprise 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of the anti-IL-23A antibody. In one embodiment, in any one of the methods above, the initial dose and the loading dose comprise 75 mg of the anti-IL-23A antibody.

In one embodiment, in any one of the methods above, the initial dose, the loading dose, the first maintenance dose and the at least one additional maintenance dose comprise 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of the anti-IL-23A antibody. In one embodiment, in any one of the methods above, the initial dose, the loading dose, the first maintenance dose and the at least one additional maintenance dose comprise 75 mg of the anti-IL-23A antibody.

In one embodiment, the present invention further provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of said anti-IL-23A antibody to the patient;
b) administering a loading dose of said anti-IL-23A antibody to the patient 4 weeks after said initial dose is administered;
c) administering a first maintenance dose of said anti-IL-23A antibody to the patient 12 weeks after said loading dose is administered; and
d) administering at least one additional maintenance dose to the patient 12 weeks after said first maintenance dose is administered.

In one embodiment, in said method, the initial dose, the loading dose and the maintenance dose comprise 150 mg of said anti-IL-23A antibody. In one embodiment, in said method, the initial dose and the loading dose comprise 300 mg of said anti-IL-23A antibody and the maintenance dose comprises 150 mg of said anti-IL-23A antibody.

In one embodiment, in said method, the initial dose and the loading dose comprise 180 mg of said anti-IL-23A antibody and the maintenance dose comprises 90 mg of said anti-IL-23A antibody. In one embodiment, in said method, the initial dose, the loading dose and the maintenance dose comprise 75 mg of said anti-IL-23A antibody.

In one embodiment, the present invention further provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of said anti-IL-23A antibody to the patient;
b) administering a loading dose of said anti-IL-23A antibody to the patient 4 weeks after said initial dose is administered;
c) administering a first maintenance dose of said anti-IL-23A antibody to the patient 8 weeks after said loading dose is administered; and
d) administering at least one additional maintenance dose to the patient 8 weeks after said first maintenance dose is administered.

In one embodiment, in said method, the initial dose, the loading dose and the maintenance dose comprise 90 mg of said anti-IL-23A antibody. In one embodiment, in said method, the initial dose, the loading dose and the maintenance dose comprise 150 mg of said anti-IL-23A antibody.

In one embodiment, the present invention further provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of said anti-IL-23A antibody to the patient;
b) administering a loading dose of said anti-IL-23A antibody to the patient 4 weeks after said initial dose is administered;
c) administering a first maintenance dose of said anti-IL-23A antibody to the patient 16 weeks after said loading dose is administered; and
d) administering at least one additional maintenance dose to the patient 16 weeks after said first maintenance dose is administered.

In one embodiment, in said method, the initial dose, the loading dose and the maintenance dose comprise 150 mg of said anti-IL-23A antibody.

In one embodiment, the present invention further provides a method for treating an inflammatory disease, in one aspect for treating psoriasis, psoriatic arthritis or axial (spinal) spondyloarthritis (ax-SpA), including ankylosing spondylitis and non-radiographic ax-SpA, comprising administering to a patient an anti-IL-23A antibody, said method comprising:
a) administering an initial dose of said anti-IL-23A antibody to the patient;
b) administering a loading dose of said anti-IL-23A antibody to the patient 4 weeks after said initial dose is administered;
c) administering a first maintenance dose of said anti-IL-23A antibody to the patient 6 weeks after said loading dose is administered; and
d) administering at least one additional maintenance dose to the patient 6 weeks after said first maintenance dose is administered.

In one embodiment, in said method, the initial dose, the loading dose and the maintenance dose comprise 150 mg of said anti-IL-23A antibody.

In one embodiment, the present invention further provides a method for treating an inflammatory disease comprising administering to a patient 15 to 300 mg, for example 15 to 250 mg, for example 90 to 180 mg, of an anti-IL-23A antibody. In one embodiment, the inflammatory disease is psoriasis, psoriatic arthritis or axial (spinal) spondyloarthritis (ax-SpA), including ankylosing spondylitis and non-radiographic ax-SpA.

In one embodiment, the method comprises administering to a patient 70 to 90 mg, 80 to 100 mg, 90 to 110 mg, 100 to 120 mg, 110 to 130 mg, 120 to 140 mg, 130 to 150 mg, 140 to 160 mg, 150 to 170 mg, 160 to 180 mg, 170 to 190 mg, 180 to 200 mg, 190 to 210 mg, 200 to 220 mg, 210 to 230 mg, 220 to 240 mg, 230 to 250 mg, 240 to 260 mg, 250 to 270 mg, 260 to 280 mg, 270 to 290 mg or 280 to 300 mg of an anti-IL-23A antibody.

In one embodiment, the method comprises administering to a patient 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of an anti-IL-23A antibody. In one embodiment, the method comprises administering to a patient 75 mg of an anti-IL-23A antibody.

In one embodiment, the anti-IL-23A antibody is administered as an initial dose, a loading dose or a maintenance dose.

In one embodiment, the present invention further provides a method for treating an inflammatory disease, in one aspect for treating psoriasis or psoriatic arthritis, comprising administering to a patient an anti-IL-23A antibody, said method comprising:
a) administering an initial dose of said anti-IL-23A antibody to the patient; and
b) administering a second dose of said anti-IL-23A antibody to the patient when the patient no longer maintains a certain PASI score, for example PASI 90, PASI 75, PASI 100 or PASI 50.

In one aspect, the initial dose and the second dose are doses as described herein.

In one embodiment, the present invention further provides a method for treating an inflammatory disease, in one aspect for treating psoriasis or psoriatic arthritis, comprising administering to a patient an anti-IL-23A antibody, said method comprising:
a) administering an initial dose of said anti-IL-23A antibody to the patient;
b) administering a loading dose of said anti-IL-23A antibody to the patient 1 to 6 weeks, for example 4 weeks, after said initial dose is administered; and
c) administering a third dose of said anti-IL-23A antibody to the patient when the patient no longer maintains a certain PASI score, for example PASI 90, PASI 75, PASI 100 or PASI 50.

In one aspect, the initial dose, the loading dose and the third dose are doses as described herein.

In one embodiment, in any one of the methods above, the anti-IL-23A antibody is Antibody A, Antibody B, Antibody C or Antibody D.

In one embodiment, in any one of the methods above, the method is for treating plaque psoriasis, for example chronic plaque psoriasis.

In one embodiment, in any one of the methods above, the method is for treating moderate to severe chronic plaque psoriasis, for example in a patient who is a candidate for systemic therapy or phototherapy.

In one embodiment, in any one of the methods above, the method is for treating moderate to severe chronic plaque psoriasis, for example in a patient who failed to respond to, or who has a contraindication to, or is intolerant to other systemic therapy including ciclosporin, methotrexate, psoralen or ultraviolet-A light (PUVA).

In one embodiment, in any one of the methods above, the method is for treating pustular psoriasis.

In one embodiment, in any one of the methods above, the method is for treating erythodermic psoriasis (also known as psoriatic erythroderma).

In one embodiment, in any one of the methods above, the method is for treating generalized pustular psoriasis (GPP). In one embodiment, the present invention provides a method for treating generalized pustular psoriasis (GPP) comprising administering to a patient an anti-IL-23A antibody, for example an anti-IL-23A antibody as described herein.

In one embodiment, in any one of the methods above, the method is for treating psoriatic arthritis, for example active psoriatic arthritis.

In one embodiment, the anti-IL-23A antibody is used alone or in combination with one or more non-biologic DMARDs (Disease-Modifying Antirheumatic Drug), for example for treating psoriatic arthritis, for example active psoriatic arthritis, for example to reduce signs and symptoms. In one embodiment, the anti-IL-23A antibody is used or indicated to inhibit the progression of structural damage, and/or improve physical function.

In one embodiment, the anti-IL-23A antibody is used alone or in combination with methotrexate (MTX), for example for the treatment of psoriatic arthritis, for example active psoriatic arthritis, for example when the response to previous non-biological DMARD therapy has been inadequate. In one aspect, the anti-IL-23A antibody is used to reduce the rate of progression of peripheral joint damage as measured by X-ray and/or to improve physical function.

In one embodiment, in any one of the methods above, the anti-IL-23A antibody is administered by subcutaneous administration.

In one aspect, in a method of the present invention, 90 - 180 mg of the anti-IL-23 antibody is administered to the patient every 6-12 weeks, for example every 8 - 12 weeks, with or without a loading dose.

In one aspect, in a method of the present invention, a first maintenance dose is administered to the patient up to 32 weeks after the administration of the initial dose. In one aspect, in a method of the present invention, the first maintenance dose is administered to the patient 28 weeks or 32 weeks after the administration of the initial dose.

In one aspect, in a method of the present invention, a first maintenance dose is administered to the patient up to 32 weeks after the administration of the loading dose. In one aspect, in a method of the present invention, the first maintenance dose is administered to the patient 28 weeks or 32 weeks after the administration of the loading dose.

In one aspect, in a method of the present invention, at least one additional maintenance dose is administered to the patient up to 32 weeks after the administration of the first maintenance dose. In one aspect, in a method of the present invention, at least one additional maintenance dose is administered to the patient 28 weeks or 32 weeks after the administration of the first maintenance dose.

In one embodiment, the present invention further provides a method for treating psoriasis, said method comprising administering Antibody A to a patient previously treated with a first agent. In one aspect, the PASI score or the sPGA score of said patient improves after the administration of Antibody A. In one aspect, the patient reaches PASI 90 after the administration of Antibody A. In one aspect, the patient reaches PASI 100 after the administration of Antibody A. In one aspect, the patient reaches a sPGA score of clear (score of 0) or almost clear (score of 1) after the administration of Antibody A. In one aspect, the patient did not reach PASI 90 before the administration of Antibody A. In one aspect, the patient did not reach PASI 75 before the administration of Antibody A. In one aspect, the patient did not reach PASI 50 before the administration of Antibody A. In one aspect, the patient did not reach a sPGA score of clear (score of 0) or almost clear (score of 1) before the administration of Antibody A. In one aspect, the first agent is a biological drug, for example a monoclonal antibody. In one aspect, the first agent is ustekinumab or adalimumab.

In one embodiment, the present invention further provides a method for treating psoriasis, said method comprising identifying a patient treated with a first agent, wherein the therapeutic effect of said first agent in said patient is insufficient to treat psoriasis; and administering Antibody A to said patient. In one aspect, the therapeutic effect of Antibody A in said patient is sufficient to treat psoriasis. In one aspect, the patient does not reach PASI 90 before the administration of Antibody A. In one aspect, the patient does not reach PASI 75 before the administration of Antibody A. In one aspect, the patient does not reach PASI 50 before the administration of Antibody A. In one aspect, the patient does not reach a sPGA score of clear (score of 0) or almost clear (score of 1) before the administration of Antibody A. In one aspect, the patient reaches PASI 90 after the administration of Antibody A. In one aspect, the patient reaches PASI 100 after the administration of Antibody A. In one aspect, the patient reaches a sPGA score of clear (score of 0) or almost clear (score of 1) after the administration of Antibody A. In one aspect, the first agent is a biological drug, for example a monoclonal antibody. In one aspect, the first agent is ustekinumab or adalimumab.

In one embodiment, the present invention further provides a method for treating psoriasis comprising switching a patient who does not fully respond to a first agent from treatment with said first agent to treatment with Antibody A. In one aspect, a patient does not fully respond when a PASI score of 90 is not reached. In one aspect, a patient does not fully respond when a PASI score of 75 is not reached. In one aspect, a patient does not fully respond when a PASI score of 50 is not reached. In one aspect, a patient does not fully respond when a sPGA score of clear (score of 0) or almost clear (score of 1) is not reached. In one aspect, the first agent is a biological drug, for example a monoclonal antibody. In one aspect, the first agent is ustekinumab or adalimumab.

In one embodiment, in any one of the methods above, the patient is an adult patient.

In one aspect, the present invention provides an anti-IL-23A antibody for use in the treatment of a disease as described herein.

In one aspect, the present invention provides an anti-IL-23A antibody for use in the treatment of a disease, for example an inflammatory disease, for example psoriasis, psoriatic arthritis or axial (spinal) spondyloarthritis (ax-SpA), including Ankylosing Spondylitis and non-radiographic ax-SpA, by administration in certain amounts and/or at certain intervals as described herein.

In one aspect, the present invention provides for the use of an anti-IL-23A antibody for the preparation of a medicament for the treatment of a disease as described herein.

In one aspect, the present invention provides for the use of an anti-IL-23A antibody for the preparation of a medicament for the treatment of a disease, for example an inflammatory disease, for example psoriasis, psoriatic arthritis or axial (spinal) spondyloarthritis (ax-SpA), including Ankylosing Spondylitis and non-radiographic ax-SpA, by administration in certain amounts and/or at certain intervals as described herein.

In one embodiment, in any one of the methods or uses above, the anti-IL-23A antibody is disclosed below.

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO: 4, 7, 8 or 9 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:4 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:7 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:8 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:9 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of any one of SEQ ID NO:10, 11, 12 or 13; and a heavy chain variable region comprising the amino acid sequence any one of SEQ ID NO:14, 15, 16 or 17.

In one embodiment, the anti-IL-23A antibody comprises a light chain variable region comprising the amino acid sequence of SEQ ID NO:11 and a heavy chain variable region comprising the amino acid sequence SEQ ID NO:14.

In one embodiment, the anti-IL-23A antibody comprises a light chain variable region comprising the amino acid sequence of SEQ ID NO:11 and a heavy chain variable region comprising the amino acid sequence SEQ ID NO:15.

In one embodiment, the anti-IL-23A antibody comprises a light chain variable region comprising the amino acid sequence of SEQ ID NO:10 and a heavy chain variable region comprising the amino acid sequence SEQ ID NO:14.

In one embodiment, the anti-IL-23A antibody comprises a light chain variable region comprising the amino acid sequence of SEQ ID NO:10 and a heavy chain variable region comprising the amino acid sequence SEQ ID NO:15.

In one embodiment, the anti-IL-23A antibody comprises the amino acid sequence SEQ ID NO:14 or 15 linked to a human IgG1, IgG2, IgG3, IgG4, IgM, IgA or IgE heavy chain constant region. In one embodiment, the anti-IL-23A antibody comprises the amino acid sequence of SEQ ID NO: 14 or 15 linked to a human IgG1 heavy chain constant region. In one embodiment, the anti-IL-23A antibody comprises the amino acid sequence of SEQ ID NO:10 or 11 linked to a human kappa or lambda light chain constant region.

In one embodiment, In one embodiment, the anti-IL-23A antibody comprises the amino acid sequence of SEQ ID NO:14 or 15 linked to a human IgG1 heavy chain constant region; and the amino acid sequence of SEQ ID NO: 10 or 11 linked to a human kappa light chain constant region.

In one embodiment, the anti-IL-23A antibody is a humanized monoclonal antibody comprising a light chain variable region comprising the amino acid sequence selected from the group consisting of any one of SEQ ID NO:10, 11, 12 and 13 and a heavy chain variable region comprising the amino acid sequence selected from the group consisting of any one of SEQ ID NO:14, 15, 16 and 17.

In one embodiment, the anti-IL-23A antibody is a humanized monoclonal antibody comprising a light chain variable region comprising the amino acid sequence of SEQ ID NO:11 and a heavy chain variable region comprising the amino acid sequence SEQ ID NO:14.

In one embodiment, the anti-IL-23A antibody is a humanized monoclonal antibody comprising a light chain variable region comprising the amino acid sequence of SEQ ID NO:11 and a heavy chain variable region comprising the amino acid sequence SEQ ID NO:15.

In one embodiment, the anti-IL-23A antibody is a humanized monoclonal antibody comprising a light chain variable region comprising the amino acid sequence of SEQ ID NO:10 and a heavy chain variable region comprising the amino acid sequence SEQ ID NO:14.

In one embodiment, the anti-IL-23A antibody is a humanized monoclonal antibody comprising a light chain variable region comprising the amino acid sequence of SEQ ID NO:10 and a heavy chain variable region comprising the amino acid sequence SEQ ID NO:15.

In one embodiment, the anti-IL-23A antibody comprises a light chain comprising the amino acid sequence of SEQ ID NO: 18 or 21 and a heavy chain comprising the amino acid sequence of SEQ ID NO:19 or 20.

In one embodiment, the anti-IL-23A antibody comprises a light chain comprising the amino acid sequence of SEQ ID NO: 18 and a heavy chain comprising the amino acid sequence of SEQ ID NO: 19.

In one embodiment, the anti-IL-23A antibody comprises a light chain comprising the amino acid sequence of SEQ ID NO: 18 and a heavy chain comprising the amino acid sequence of SEQ ID NO:20.

In one embodiment, the anti-IL-23A antibody comprises a light chain comprising the amino acid sequence of SEQ ID NO:21 and a heavy chain comprising the amino acid sequence of SEQ ID NO: 19.

In one embodiment, the anti-IL-23A antibody comprises a light chain comprising the amino acid sequence of SEQ ID NO: 21 and a heavy chain comprising the amino acid sequence of SEQ ID NO: 20.

In one embodiment, the anti-IL-23A antibody is Antibody A, Antibody B, Antibody C or Antibody D.

In one embodiment, the anti-IL-23A antibody is as disclosed in WO2007/005955, WO2007/024846, WO2007/027714, WO2007/076524, WO2008/103432 or WO2012/061448.

In one embodiment, the present invention provides a method for detecting the presence or absence of a beneficial response in a patient after administration of an IL-23A antagonist, comprising: a) obtaining a biological sample from the patient; b) measuring in said sample the level of expression of one or more genes; c) comparing the level in b) to the level of expression of the one or more genes in a control; and d) determining whether or not the difference in levels between the sample and the control reflects a beneficial response in the patient, wherein the one or more genes is one or more genes described herein. In one aspect, the patient suffers from psoriasis. In one aspect, the control is from one or more subjects that do not suffer from psoriasis. In one aspect, the control is from one or more subjects treated with another agent, for example an anti-IL-12/IL-23 antibody, for example ustekinumab. In one aspect, the IL-23A antagonist is an anti-IL-23A antibody or an antigen binding fragment thereof, for example Antibody A, Antibody B, Antibody C or Antibody D.

In one embodiment, the anti-IL-23A antibody modulates the genes as described in Example 5. In one embodiment, the anti-IL-23A antibody modulates the pathways as described in Example 5. In one embodiment, the anti-IL-23A antibody modulates one of the genes as described in Example 5. In one embodiment, the anti-IL-23A antibody modulates one of the pathways as described in Example 5.

### BRIEF DESCRIPTION OF THE FIGURES

Figure 1: Rate of patients with >50% reductions from baseline pain-VAS (%).
Figure 2: Achievement of PASI 50 over time (observed).
Figure 3: Achievement of PASI 75 over time (observed).
Figure 4: Achievement of PASI 90 over time (observed).
Figure 5: Achievement of PASI 100 over time (observed).

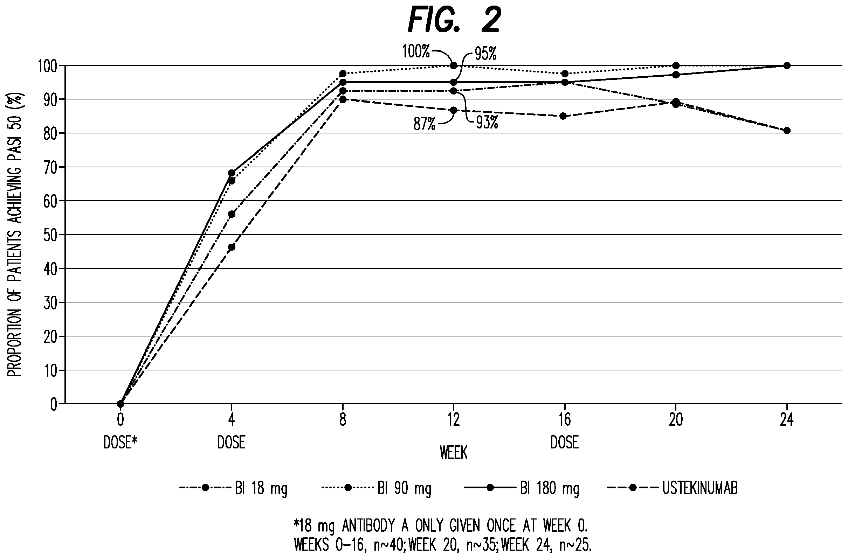

Antibody A is also referred to as "BI" in Figures 2 to 5.

### DETAILED DESCRIPTION

The p19 subunit of IL-23 (also referred to herein as "IL-23A", "IL-23p19" and "p19 subunit") is a 189 amino acid polypeptide containing a 21 aa leader sequence (Oppmann et al. Immunity 13:715 (2000), SEQ ID NO: 22). The biological activity of the molecule is only detected when it is partnered with the IL-12p40 subunit to form IL-23. IL-23 is predominantly expressed by activated dendritic cells (DCs) and phagocytic cells. The receptor for IL-23 was found to be composed of the IL-12R□ 1 subunit of IL-12 receptor partnered with a unique subunit called IL-23R (Parham et al. J. Immunol. 168:5699 (2002)). Expression of the receptor is detected primarily on memory T cells and NK cells. Thus, expression of this cytokine:receptor pair appears to be restricted to specific populations of immune cells. While it was first thought that IL-12 and IL-23 would share many functions, the data has shown the picture to be different. Whereas IL-12 has a predominant role in the production of Th1 cells, IL-23 was found to be critically involved in the production and maintenance of a recently recognized Th cell subset termed Th17 (Kikly et al. Curr. Opin. Immunol. 18:670 (2006), Kastelein et al. Ann. Rev. Immunol. 25:221 (2007)). These cells produce IL-17A, IL-17F, IL-22 and other pro-inflammatory cytokines such as IL-6 and TNF-α. As described below, animal model studies on the role of these Th17 cells show their importance as a driving force in chronic inflammation and autoimmunity.

SEQ ID NO: 22:
mlgsravmll lllpwtaqgr avpggsspaw tqcqqlsqkl ctlawsahpl vghmdlreeg deettndvph iqcgdgcdpq glrdnsqfcl qrihqglify ekllgsdift gepsllpdsp vgqlhasllg lsqllqpegh hwetqqipsl spsqpwqrll lrfkilrslq afvavaarvf ahgaatlsp

In one aspect, the present invention provides methods for the treatment of IL-23A related diseases. In one aspect, the present invention provides methods for treating a disease, for example an inflammatory disease, in particular methods comprising administering an anti-IL-23A antibody to a patient in certain amounts and/or at certain intervals. In one aspect, a method of the present invention is for the treatment of psoriasis or psoriatic arthritis. In one aspect, a method of the present invention is for the treatment of Ankylosing Spondylitis (AS). In one aspect, a method of the present invention is for the treatment axial (spinal) spondyloarthritis (ax-SpA), for example non-radiographic ax-SpA.

In one aspect, the present invention provides an anti-IL-23A antibody for use in the treatment of a disease, for example an inflammatory disease, for example psoriasis, psoriatic arthritis or axial (spinal) spondyloarthritis (ax-SpA), including Ankylosing Spondylitis and non-radiographic ax-SpA, by administration in certain amounts and/or at certain intervals as described herein.

In one aspect, the present invention provides for the use of an anti-IL-23A antibody for the preparation of a medicament for the treatment of a disease, for example an inflammatory disease, for example psoriasis, psoriatic arthritis or axial (spinal) spondyloarthritis (ax-SpA), including Ankylosing Spondylitis and non-radiographic ax-SpA, by administration in certain amounts and/or at certain intervals as described herein.

In one aspect, a method of the present invention comprises administering to the patient an initial dose of the anti-IL-23A antibody followed by the administration of one or more maintenances doses of the anti-IL-23A antibody. Optionally, a loading dose of the anti-IL-23A antibody is administered to the patient between the administration of the initial dose and the administration of the first maintenance dose.

In one aspect, in a method of the instant invention the interval between the administration of the initial dose and the first maintenance dose is 4 to 24 weeks, for example 4 to 16 weeks, for example 4 to 12 weeks, for example 4, 6, 8, 12, 16, 20 or 24 weeks. In another aspect, the interval between the administration of the first maintenance dose and the subsequent maintenance doses is 4 to 24 weeks, for example 4 to 16 weeks, for example 4 to 12 weeks, for example 4, 6, 8, 12, 16, 20 or 24 weeks.

In one aspect, in a method of the instant invention the interval between the administration of the initial dose and the first maintenance dose is 8 to 24 weeks, for example 8 to 16 weeks, for example 8 to 12 weeks, for example 6 to 24 weeks, for example 6 to 16 weeks, for example 6 to 12 weeks, for example 6, 8, 12, 16, 20 or 24 weeks. In another aspect, the interval between the administration of the first maintenance dose and the subsequent maintenance doses is 8 to 24 weeks, for example 8 to 16 weeks, for example 8 to 12 weeks, for example 6 to 24 weeks, for example 6 to 16 weeks, for example 6 to 12 weeks, for example 6, 8, 12, 16, 20 or 24 weeks.

In one aspect, in a method of the instant invention, the interval between the administration of the initial dose and the first maintenance dose and the interval between the administration of the first maintenance dose and the subsequent maintenance doses are the same, for example 4 to 24 weeks, for example 4 to 16 weeks, for example 4 to 12 weeks, for example 4, 6, 8, 12, 16, 20 or 24 weeks.

In one aspect, in a method of the instant invention, the interval between the administration of the initial dose and the first maintenance dose and the interval between the administration of the first maintenance dose and the subsequent maintenance doses are the same, for example 8 to 24 weeks, for example 8 to 16 weeks, for example 8 to 12 weeks, for example 6 to 24 weeks, for example 6 to 16 weeks, for example 6 to 12 weeks, for example 6, 8, 12, 16, 20 or 24 weeks.

In one aspect, in a method of the instant invention, a loading dose of the anti-IL-23A antibody is administered to the patient between the administration of the initial dose and the administration of the first maintenance doses.

In one aspect, the loading dose is administered to the patient 1, 2, 3, 4, 5 or 6 weeks after the administration of the initial dose. In one aspect, in a method of the instant invention the interval between the administration of the loading dose and the first maintenance dose is 8 to 24 weeks, for example 8 to 16 weeks, for example 8 to 12 weeks, for example 6 to 24 weeks, for example 6 to 16 weeks, for example 6 to 12 weeks, for example 6, 8, 12, 16, 20 or 24 weeks. In another aspect, the interval between the administration of the first maintenance dose and the subsequent maintenance doses is 8 to 24 weeks, for example 8 to 16 weeks, for example 8 to 12 weeks, for example 6 to 24 weeks, for example 6 to 16 weeks, for example 6 to 12 weeks, for example 6, 8, 12, 16, 20 or 24 weeks.

In one aspect, in a method of the instant invention, the interval between the administration of the loading dose and the first maintenance dose and the interval between the administration of the first maintenance dose and the subsequent maintenance doses are the same, for example 8 to 24 weeks, for example 8 to 16 weeks, for example 8 to 12 weeks, for example 6 to 24 weeks, for example 6 to 16 weeks, for example 6 to 12 weeks, for example 6, 8, 12, 16, 20 or 24 weeks.

In one aspect, the amount of anti-IL-23A antibody in the initial dose and in the maintenance dose is the same. In one aspect, the amount of anti-IL-23A antibody in the initial dose and in the loading dose is the same. In one aspect, the amount of anti-IL-23A antibody in the loading dose and in the maintenance dose is the same. In one aspect, the amount of anti-IL-23A antibody in the initial dose, in the loading dose and in the maintenance dose is the same.

In one aspect, the amount of anti-IL-23A antibody in the maintenance doses is lower than the amount of anti-IL-23A antibody in the initial dose and/or loading dose. In one aspect, the amount of anti-IL-23A antibody in the initial dose is twice the amount of anti-IL-23A antibody in the maintenance dose, for example in the absence of a loading dose. In one aspect, the amount of anti-IL-23A antibody in the initial dose and in the loading dose is twice the amount of anti-IL-23A antibody in the maintenance dose.

In one aspect, in a method of the present invention, the initial dose comprises 15 to 300 mg, for example 15 to 250 mg of the anti-IL-23A antibody. In another aspect, the maintenance doses comprise 15 to 300 mg, for example 15 to 250 mg of the anti-IL-23A antibody. In another aspect, if a loading dose is administered, such loading dose comprises 15 to 300 mg, for example 15 to 250 mg of the anti-IL-23A antibody.

In a further aspect, an initial dose, a loading dose or a maintenance dose according to the present invention comprises 70 to 90 mg, 80 to 100 mg, 90 to 110 mg, 100 to 120 mg, 110 to 130 mg, 120 to 140 mg, 130 to 150 mg, 140 to 160 mg, 150 to 170 mg, 160 to 180 mg, 170 to 190 mg, 180 to 200 mg, 190 to 210 mg, 200 to 220 mg, 210 to 230 mg, 220 to 240 mg, 230 to 250 mg, 240 to 260 mg, 250 to 270 mg, 260 to 280 mg, 270 to 290 mg or 280 to 300 mg of the anti-IL-23A antibody.

In a further aspect, an initial dose, a loading dose or a maintenance dose according to the present invention comprises 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of the anti-IL-23A antibody. In a further aspect, an initial dose, a loading dose or a maintenance dose according to the present invention comprises 75 mg of the anti-IL-23A antibody.

In a further aspect, the present invention provides a method for treating an inflammatory disease, said method comprising administering to a patient 15 to 250 mg of an anti-IL-23A antibody. In one aspect, 70 to 90 mg, 80 to 100 mg, 90 to 110 mg, 100 to 120 mg, 110 to 130 mg, 120 to 140 mg, 130 to 150 mg, 140 to 160 mg, 150 to 170 mg, 160 to 180 mg, 170 to 190 mg, 180 to 200 mg, 190 to 210 mg, 200 to 220 mg, 210 to 230 mg, 220 to 240 mg, 230 to 250 mg, 240 to 260 mg, 250 to 270 mg, 260 to 280 mg, 270 to 290 mg or 280 to 300 mg of the anti-IL-23A antibody is administered to the patient. In one aspect, 18, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of the anti-IL-23A antibody is administered to the patient. In one aspect, 75 mg of the anti-IL-23A antibody is administered to the patient. In one aspect, the disease is psoriasis or psoriatic arthritis. In one aspect, the disease is axial (spinal) spondyloarthritis (ax-SpA), including ankylosing spondylitis and non-radiographic ax-SpA.

In one aspect, in a method of the present invention, 90 - 180 mg of the anti-IL-23 antibody is administered to the patient every 6-12 weeks, for example every 8 - 12 weeks, with or without a loading dose.

Representative examples of doses and dose regimens according to the present invention are disclosed in Table A.

**Table A: doses and dose regimens**

| Initial dose (mg) | Loading dose (mg, 4 weeks after initial dose) | Maintenance dose (mg) | Frequency of maintenance doses | Alternative frequencies of maintenance doses |
|---|---|---|---|---|
| 90 | x | 90 | Every 12 weeks | Every 6, 8, 16, 20 or 24 weeks |
| 90 | 90 | 90 | Every 12 weeks | Every 6, 8, 16, 20 or 24 weeks |
| 180 | x | 180 | Every 12 weeks | Every 6, 8, 16, 20 or 24 weeks |
| 180 | 180 | 180 | Every 12 weeks | Every 6, 8, 16, 20 or 24 weeks |
| 150 | x | 150 | Every 12 weeks | Every 6, 8, 16, 20 or 24 weeks |
| 150 | 150 | 150 | Every 12 weeks | Every 6, 8, 16, 20 or 24 weeks |
| 140 | x | 140 | Every 12 weeks | Every 6, 8, 16, 20 or 24 weeks |
| 140 | 140 | 140 | Every 12 weeks | Every 6, 8, 16, 20 or 24 weeks |
| 130 | x | 130 | Every 12 weeks | Every 6, 8, 16, 20 or 24 weeks |
| 130 | 130 | 130 | Every 12 weeks | Every 6, 8, 16, 20 or 24 weeks |
| 120 | x | 120 | Every 12 weeks | Every 6, 8, 16, 20 or 24 weeks |
| 120 | 120 | 120 | Every 12 weeks | Every 6, 8, 16, 20 or 24 weeks |
| 110 | x | 110 | Every 12 weeks | Every 6, 8, 16, 20 or 24 weeks |
| 110 | 110 | 110 | Every 12 weeks | Every 6, 8, 16, 20 or 24 weeks |
| 100 | x | 100 | Every 12 weeks | Every 6, 8, 16, 20 or 24 weeks |
| 100 | 100 | 100 | Every 12 weeks | Every 6, 8, 16, 20 or 24 weeks |
| 160 | x | 160 | Every 12 weeks | Every 6, 8, 16, 20 or 24 weeks |
| 160 | 160 | 160 | Every 12 weeks | Every 6, 8, 16, 20 or 24 weeks |
| 170 | x | 170 | Every 12 weeks | Every 6, 8, 16, 20 or 24 weeks |
| 170 | 170 | 170 | Every 12 weeks | Every 6, 8, 16, 20 or 24 weeks |
| 190 | x | 190 | Every 12 weeks | Every 6, 8, 16, 20 or 24 weeks |
| 190 | 190 | 190 | Every 12 weeks | Every 6, 8, 16, 20 or 24 weeks |
| 200 | x | 200 | Every 12 weeks | Every 6, 8, 16, 20 or 24 weeks |
| 200 | 200 | 200 | Every 12 weeks | Every 6, 8, 16, 20 or 24 weeks |
| 210 | 210 | 210 | Every 12 weeks | Every 6, 8, 16, 20 or 24 weeks |
| 220 | 220 | 220 | Every 12 weeks | Every 6, 8, 16, 20 or 24 weeks |
| 230 | 230 | 230 | Every 12 weeks | Every 6, 8, 16, 20 or 24 weeks |
| 240 | 240 | 240 | Every 12 weeks | Every 6, 8, 16, 20 or 24 weeks |
| 250 | 250 | 250 | Every 12 weeks | Every 6, 8, 16, 20 or 24 weeks |
| 260 | 260 | 260 | Every 12 weeks | Every 6, 8, 16, 20 or 24 weeks |
| 270 | 270 | 270 | Every 12 weeks | Every 6, 8, 16, 20 or 24 weeks |
| 280 | 280 | 280 | Every 12 weeks | Every 6, 8, 16, 20 or 24 weeks |
| 290 | 290 | 290 | Every 12 weeks | Every 6, 8, 16, 20 or 24 weeks |
| 300 | 300 | 300 | Every 12 weeks | Every 6, 8, 16, 20 or 24 weeks |

| | | | | |
|---|---|---|---|---|
| x : no loading dose administered. | | | | |

In Table A when a loading dose is administered, it is indicated as being administered 4 weeks after the initial dose.

It is also within the scope of the instant invention to administer the loading dose 1 week after the initial dose for each of the doses and frequencies of maintenance doses identified in the above table.

It is also within the scope of the instant invention to administer the loading dose 2 week after the initial dose for each of the doses and frequencies of maintenance doses identified in the above table.

It is also within the scope of the instant invention to administer the loading dose 3 week after the initial dose for each of the doses and frequencies of maintenance doses identified in the above table.

It is also within the scope of the instant invention to administer the loading dose 5 week after the initial dose for each of the doses and frequencies of maintenance doses identified in the above table.

It is also within the scope of the instant invention to administer the loading dose 6 week after the initial dose for each of the doses and frequencies of maintenance doses identified in the above table.

For example, in the context of the present invention, if no loading dose is administered to the patient, and with a frequency of administration of the maintenance doses of 12 weeks, the initial dose is administered to the patient at week 0, followed by a further administration at week 12 (first maintenance dose), then at week 24 (second maintenance dose) and so on at dosing intervals of 12 weeks. In one aspect, the initial dose and the maintenance doses comprise 150 mg of the anti-IL-23A antibody. In one aspect, the initial dose and the maintenance doses comprise 75 mg of the anti-IL-23A antibody.

For example, in the context of the present invention, if a loading dose is administered to the patient, and with a frequency of administration of the maintenance doses of 12 weeks, the initial dose is administered to the patient at week 0, followed by a further administration at week 4 (loading dose), then at week 16 (first maintenance dose), at week 28 (second maintenance dose) and so on at dosing intervals of 12 weeks. In one aspect, the initial dose, the loading dose and the maintenance doses comprise 150 mg of the anti-IL-23A antibody. In one aspect, the initial dose and the loading dose comprise 300 mg of the anti-IL-23A antibody and the maintenance doses comprise 150 mg of the anti-IL-23A antibody. In one aspect, the initial dose and the loading dose comprise 180 mg of the anti-IL-23A antibody and the maintenance doses comprise 90 mg of the anti-IL-23A antibody. In one aspect, the initial dose, the loading dose and the maintenance doses comprise 75 mg of the anti-IL-23A antibody.

For example, in the context of the present invention, if no loading dose is administered to the patient, and with a frequency of administration of the maintenance doses of 8 weeks, the initial dose is administered to the patient at week 0, followed by a further administration at week 8 (first maintenance dose), then at week 16 (second maintenance dose) and so on at dosing intervals of 8 weeks. In one aspect, the initial dose and the maintenance doses comprise 90 mg of the anti-IL-23A antibody. In one aspect, the initial dose and the maintenance doses comprise 150 mg of the anti-IL-23A antibody.

For example, in the context of the present invention, if a loading dose is administered to the patient, and with a frequency of administration of the maintenance doses of 8 weeks, the initial dose is administered to the patient at week 0, followed by a further administration at week 4 (loading dose), then at week 12 (first maintenance dose), at week 20 (second maintenance dose) and so on at dosing intervals of 8 weeks. In one aspect, the initial dose, the loading dose and the maintenance doses comprise 90 mg of the anti-IL-23A antibody. In one aspect, the initial dose, the loading dose and the maintenance doses comprise 150 mg of the anti-IL-23A antibody.

For example, in the context of the present invention, if no loading dose is administered to the patient, and with a frequency of administration of the maintenance doses of 16 weeks, the initial dose is administered to the patient at week 0, followed by a further administration at week 16 (first maintenance dose), then at week 32 (second maintenance dose) and so on at dosing intervals of 16 weeks. In one aspect, the initial dose and the maintenance doses comprise 150 mg of the anti-IL-23A antibody.

For example, in the context of the present invention, if a loading dose is administered to the patient, and with a frequency of administration of the maintenance doses of 16 weeks, the initial dose is administered to the patient at week 0, followed by a further administration at week 4 (loading dose), then at week 20 (first maintenance dose), at week 36 (second maintenance dose) and so on at dosing intervals of 16 weeks. In one aspect, the initial dose, the loading dose and the maintenance doses comprise 150 mg of the anti-IL-23A antibody.

For example, in the context of the present invention, if no loading dose is administered to the patient, and with a frequency of administration of the maintenance doses of 6 weeks, the initial dose is administered to the patient at week 0, followed by a further administration at week 6 (first maintenance dose), then at week 12 (second maintenance dose) and so on at dosing intervals of 6 weeks. In one aspect, the initial dose and the maintenance doses comprise 150 mg of the anti-IL-23A antibody.

For example, in the context of the present invention, if a loading dose is administered to the patient, and with a frequency of administration of the maintenance doses of 6 weeks, the initial dose is administered to the patient at week 0, followed by a further administration at week 4 (loading dose), then at week 10 (first maintenance dose), at week 16 (second maintenance dose) and so on at dosing intervals of 6 weeks. In one aspect, the initial dose, the loading dose and the maintenance doses comprise 150 mg of the anti-IL-23A antibody.

For example, in the context of the present invention, if the frequency of administration of the maintenance doses is 4 weeks, the initial dose is administered to the patient at week 0, followed by a further administration at week 4 (first maintenance dose), then at week 8 (second maintenance dose), at week 12 (third maintenance dose) and so on at dosing intervals of 4 weeks. In one aspect, the initial dose and the maintenance doses comprise 150 mg of the anti-IL-23A antibody.

In a further aspect, in a method according to the present invention, an initial dose of an anti-IL-23 antibody is administered to the patient. A second dose of the anti-IL-23 antibody is not administered to the patient as long as the therapeutic benefit of the initial dose is maintained, for example as assessed by PASI score, for example PASI 90, PASI 75, PASI 100 or PASI 50, but a second dose is administered to the patient when the disease severity increases above a certain level, for example when the patient no longer maintains a certain PASI score, e.g. PASI 90, PASI 75, PASI 100 or PASI 50. The initial dose and the second dose of the anti-IL-23 antibody are for example doses as described herein. In one aspect, the patient is monitored after the initial dose is administered, for example by assessing the PASI score of the patient. In one aspect, a third dose of the anti-IL-23 antibody is administered to the patient when the patient no longer maintains a certain PASI score, e.g. PASI 90, PASI 75, PASI 100 or PASI 50, after the administration of the second dose. The third dose of the anti-IL-23 antibody is for example a dose as described herein, for example the same dose as the second dose. In one aspect, the patient is monitored after the second dose is administered, for example by assessing the PASI score of the patient. In a further aspect, at least one additional dose is administered to the patient in such a way.

In a further aspect, in a method according to the present invention, a loading dose of the anti-IL-23 antibody is administered to the patient after the administration of the initial dose, for example after a time period as described herein. A third dose of the anti-IL-23 antibody is not administered to the patient as long as the therapeutic benefit of the initial dose is maintained, for example as assessed by PASI score, for example PASI 90, PASI 75, PASI 100 or PASI 50, but a third dose is administered to the patient when the disease severity increases above a certain level, for example when the patient no longer maintains a certain PASI score, e.g. PASI 90, PASI 75, PASI 100 or PASI 50. In one aspect, the loading dose of the anti-IL-23 antibody is a dose as described herein. In one aspect, a fourth dose of the anti-IL-23 antibody is administered to the patient when the patient no longer maintains a certain PASI score, e.g. PASI 90, PASI 75, PASI 100 or PASI 50, after administration of the third dose. The fourth dose of the anti-IL-23 antibody is for example a dose as described herein, for example the same dose as the third dose. In one aspect, the patient is monitored after the third dose is administered, for example by assessing the PASI score of the patient. In a further aspect, at least one additional dose is administered to the patient in such a way.

Accordingly, In one embodiment, the present invention further provides a method for treating an inflammatory disease, in one aspect for treating psoriasis or psoriatic arthritis, comprising administering to a patient an anti-IL-23A antibody, said method comprising:
a) administering an initial dose of said anti-IL-23A antibody to the patient; and
b) administering a second dose of said anti-IL-23A antibody to the patient when the patient no longer maintains PASI 90, PASI 75, PASI 100 or PASI 50.

In one embodiment, the present invention further provides a method for treating an inflammatory disease, in one aspect for treating psoriasis or psoriatic arthritis, comprising administering to a patient an anti-IL-23A antibody, said method comprising:
a) administering an initial dose of said anti-IL-23A antibody to the patient; and
b) administering a second dose of said anti-IL-23A antibody to the patient when the patient no longer maintains a sPGA score of clear (score of 0) or almost clear (score of 1).

In one aspect, in a method of the present invention, a first maintenance dose is administered to the patient up to 32 weeks after the administration of the initial dose. In one aspect, in a method of the present invention, the first maintenance dose is administered to the patient 28 weeks or 32 weeks after the administration of the initial dose.

In one aspect, in a method of the present invention, a first maintenance dose is administered to the patient up to 32 weeks after the administration of the loading dose. In one aspect, in a method of the present invention, the first maintenance dose is administered to the patient 28 weeks or 32 weeks after the administration of the loading dose.

In one aspect, in a method of the present invention, at least one additional maintenance dose is administered to the patient up to 32 weeks after the administration of the first maintenance dose. In one aspect, in a method of the present invention, at least one additional maintenance dose is administered to the patient 28 weeks or 32 weeks after the administration of the first maintenance dose.

In one aspect, the amount of anti-IL-23A antibody in the second and following doses is lower than the amount of anti-IL-23A antibody in the initial dose. In one aspect, the amount of anti-IL-23A antibody in the initial dose is twice the amount of anti-IL-23A antibody in the second and following doses.

In one embodiment, the present invention further provides a method for treating an inflammatory disease, in one aspect for treating psoriasis or psoriatic arthritis, comprising administering to a patient an anti-IL-23A antibody, said method comprising:
a) administering an initial dose of said anti-IL-23A antibody to the patient;
b) administering a loading dose of said anti-IL-23A antibody to the patient 1 to 6 weeks, for example 4 weeks, after said initial dose is administered; and
c) administering a third dose of said anti-IL-23A antibody to the patient when the patient no longer maintains PASI 90, PASI 75, PASI 100 or PASI 50.

In one aspect, the amount of anti-IL-23A antibody in the third and following doses is lower than the amount of anti-IL-23A antibody in the initial dose and/or loading dose. In one aspect, the amount of anti-IL-23A antibody in the initial dose is twice the amount of anti-IL-23A antibody in the third and following doses. In one aspect, the amount of anti-IL-23A antibody in the initial dose and in the loading dose is twice the amount of anti-IL-23A antibody in the third and following doses.

In one embodiment, an anti-IL-23A antibody in any one of the methods above is disclosed herein.

In one aspect, in any one of the methods above, a pharmaceutical composition comprising an anti-IL-23A antibody is administered to the patient. In one aspect, formulation 2 disclosed in Example 4 comprising an anti-IL-23A antibody, for example Antibody A, Antibody B, Antibody C or Antibody D is administered to the patient. In one aspect, formulation 3 disclosed in Example 4 comprising an anti-IL-23A antibody, for example Antibody A, Antibody B, Antibody C or Antibody D is administered to the patient.

In one aspect, the anti-IL-23A antibody is a humanized antibody. In one aspect, the anti-IL-23A antibody is a monoclonal antibody. In one aspect, the anti-IL-23A antibody is a full length antibody. In one aspect, the anti-IL-23A antibody is a humanized monoclonal antibody, for example a full length humanized monoclonal antibody.

An antibody described herein recognizes specific "IL-23A antigen epitope" or " IL-23A epitope". As used herein these terms refer to a molecule (e.g., a peptide) or a fragment of a molecule capable of immunoreactivity with an anti-IL-23A antibody and, for example, include an IL-23A antigenic determinant recognized by any of the antibodies having a light chain/heavy chain sequence combination of SEQ ID NO:11/14, 11/15, 10/14 or 10/15.

The generalized structure of antibodies or immunoglobulin is well known to those of skill in the art. These molecules are heterotetrameric glycoproteins, typically of about 150,000 daltons, composed of two identical light (L) chains and two identical heavy (H) chains and are typically referred to as full length antibodies. Each light chain is covalently linked to a heavy chain by one disulfide bond to form a heterodimer, and the heterotrameric molecule is formed through a covalent disulfide linkage between the two identical heavy chains of the heterodimers. Although the light and heavy chains are linked together by one disulfide bond, the number of disulfide linkages between the two heavy chains varies by immunoglobulin isotype. Each heavy and light chain also has regularly spaced intrachain disulfide bridges. Each heavy chain has at the amino-terminus a variable domain (V_{H}), followed by three or four constant domains (C_{H1}, C_{H2}, C_{H3}, and C_{H4}), as well as a hinge region between C_{H1} and C_{H2}. Each light chain has two domains, an amino-terminal variable domain (V_{L}) and a carboxy-terminal constant domain (C_{L}). The V_{L} domain associates non-covalently with the V_{H} domain, whereas the C_{L} domain is commonly covalently linked to the C_{H1} domain via a disulfide bond. Particular amino acid residues are believed to form an interface between the light and heavy chain variable domains (Chothia et al., 1985, J. Mol. Biol. 186:651-663). Variable domains are also referred herein as variable regions.

Certain domains within the variable domains differ extensively between different antibodies i.e., are "hypervariable." These hypervariable domains contain residues that are directly involved in the binding and specificity of each particular antibody for its specific antigenic determinant. Hypervariability, both in the light chain and the heavy chain variable domains, is concentrated in three segments known as complementarity determining regions (CDRs) or hypervariable loops (HVLs). CDRs are defined by sequence comparison in Kabat et al., 1991, In: Sequences of Proteins of Immunological Interest, 5th Ed. Public Health Service, National Institutes of Health, Bethesda, Md., whereas HVLs (also referred herein as CDRs) are structurally defined according to the three-dimensional structure of the variable domain, as described by Chothia and Lesk, 1987, J. Mol. Biol. 196: 901-917. These two methods result in slightly different identifications of a CDR. As defined by Kabat, CDR-L1 is positioned at about residues 24-34, CDR-L2, at about residues 50-56, and CDR-L3, at about residues 89-97 in the light chain variable domain; CDR-H1 is positioned at about residues 31-35, CDR-H2 at about residues 50-65, and CDR-H3 at about residues 95-102 in the heavy chain variable domain. The exact residue numbers that encompass a particular CDR will vary depending on the sequence and size of the CDR. Those skilled in the art can routinely determine which residues comprise a particular CDR given the variable region amino acid sequence of the antibody. The CDR1, CDR2, CDR3 of the heavy and light chains therefore define the unique and functional properties specific for a given antibody.

The three CDRs within each of the heavy and light chains are separated by framework regions (FR), which contain sequences that tend to be less variable. From the amino terminus to the carboxy terminus of the heavy and light chain variable domains, the FRs and CDRs are arranged in the order: FR1, CDR1, FR2, CDR2, FR3, CDR3, and FR4. The largely β-sheet configuration of the FRs brings the CDRs within each of the chains into close proximity to each other as well as to the CDRs from the other chain. The resulting conformation contributes to the antigen binding site (see Kabat et al., 1991, NIH Publ. No. 91-3242, Vol. I, pages 647-669), although not all CDR residues are necessarily directly involved in antigen binding.

FR residues and Ig constant domains are not directly involved in antigen binding, but contribute to antigen binding and/or mediate antibody effector function. Some FR residues are thought to have a significant effect on antigen binding in at least three ways: by noncovalently binding directly to an epitope, by interacting with one or more CDR residues, and by affecting the interface between the heavy and light chains. The constant domains are not directly involved in antigen binding but mediate various Ig effector functions, such as participation of the antibody in antibody dependent cellular cytotoxicity (ADCC), complement dependent cytotoxicity (CDC) and antibody dependent cellular phagocytosis (ADCP).

The light chains of vertebrate immunoglobulins are assigned to one of two clearly distinct classes, kappa (κ) and lambda (λ), based on the amino acid sequence of the constant domain. By comparison, the heavy chains of mammalian immunoglobulins are assigned to one of five major classes, according to the sequence of the constant domains: IgA, IgD, IgE, IgG, and IgM. IgG and IgA are further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively. The subunit structures and three-dimensional configurations of the classes of native immunoglobulins are well known.

The terms, "antibody", "anti-IL-23A antibody", "anti-IL-23p19 antibody", "humanized anti-IL-23A antibody", "humanized anti-IL-23p19 antibody", "humanized anti-IL-23A epitope antibody", humanized anti-IL-2319 epitope antibody", "variant humanized anti-IL-23A epitope antibody" and "variant humanized anti-IL-23p19 epitope antibody" specifically encompass monoclonal antibodies (including full length monoclonal antibodies), polyclonal antibodies, and antibody fragments such as variable domains and other portions of antibodies that exhibit a desired biological activity, e.g., IL-23A binding. The term "monoclonal antibody" (mAb) refers to an antibody that is highly specific, being directed against a single antigenic determinant, an "epitope". Therefore, the modifier "monoclonal" is indicative of antibodies directed to the identical epitope and is not to be construed as requiring production of the antibody by any particular method. It should be understood that monoclonal antibodies can be made by any technique or methodology known in the art; including e.g., the hybridoma method (Kohler et al., 1975, Nature 256:495), or recombinant DNA methods known in the art (see, e.g., U.S. Pat. No. 4,816,567), or methods of isolation of monoclonal recombinantly produced using phage antibody libraries, using techniques described in Clackson et al., 1991, Nature 352: 624-628, and Marks et al., 1991, J. Mol. Biol. 222: 581-597.

The term "monomer" refers to a homogenous form of an antibody. For example, for a full-length antibody, monomer means a monomeric antibody having two identical heavy chains and two identical light chains.

Chimeric antibodies consist of the heavy and light chain variable regions of an antibody from one species (e.g., a non-human mammal such as a mouse) and the heavy and light chain constant regions of another species (e.g., human) antibody and can be obtained by linking the DNA sequences encoding the variable regions of the antibody from the first species (e.g., mouse) to the DNA sequences for the constant regions of the antibody from the second (e.g. human) species and transforming a host with an expression vector containing the linked sequences to allow it to produce a chimeric antibody. Alternatively, the chimeric antibody also could be one in which one or more regions or domains of the heavy and/or light chain is identical with, homologous to, or a variant of the corresponding sequence in a monoclonal antibody from another immunoglobulin class or isotype, or from a consensus or germline sequence. Chimeric antibodies can include fragments of such antibodies, provided that the antibody fragment exhibits the desired biological activity of its parent antibody, for example binding to the same epitope (see, e.g., U.S. Pat. No. 4,816,567; and Morrison et al., 1984, Proc. Natl. Acad. Sci. USA 81: 6851-6855).

The terms, "antibody fragment", "anti-IL-23A antibody fragment", "anti-IL-23A epitope antibody fragment", "humanized anti-IL-23A antibody fragment", "humanized anti-IL-23A epitope antibody fragment", "variant humanized anti-IL-23A epitope antibody fragment" refer to a portion of a full length anti-IL-23A antibody, in which a variable region or a functional capability is retained, for example, specific IL-23A epitope binding. Examples of antibody fragments include, but are not limited to, a Fab, Fab', F(ab')₂, Fd, Fv, scFv and scFv-Fc fragment.

Full length antibodies can be treated with enzymes such as papain or pepsin to generate useful antibody fragments. Papain digestion is used to produces two identical antigen-binding antibody fragments called "Fab" fragments, each with a single antigen-binding site, and a residual "Fc" fragment. The Fab fragment also contains the constant domain of the light chain and the C_{H1} domain of the heavy chain. Pepsin treatment yields a F(ab')₂ fragment that has two antigen-binding sites and is still capable of cross-linking antigen.

Fab' fragments differ from Fab fragments by the presence of additional residues including one or more cysteines from the antibody hinge region at the C-terminus of the C_{H1} domain. F(ab')₂ antibody fragments are pairs of Fab' fragments linked by cysteine residues in the hinge region. Other chemical couplings of antibody fragments are also known.

"Fv" fragment contains a complete antigen-recognition and binding site consisting of a dimer of one heavy and one light chain variable domain in tight, non-covalent association. In this configuration, the three CDRs of each variable domain interact to define an antigen-biding site on the surface of the V_{H}-V_{L} dimer. Collectively, the six CDRs confer antigen-binding specificity to the antibody.

A "single-chain Fv" or "scFv" antibody fragment is a single chain Fv variant comprising the V_{H} and V_{L} domains of an antibody where the domains are present in a single polypeptide chain. The single chain Fv is capable of recognizing and binding antigen. The scFv polypeptide may optionally also contain a polypeptide linker positioned between the V_{H} and V_{L} domains in order to facilitate formation of a desired three-dimensional structure for antigen binding by the scFv (see, e.g., Pluckthun, 1994, In The Pharmacology of monoclonal Antibodies, Vol. 113, Rosenburg and Moore eds., Springer-Verlag, New York, pp. 269-315).

A "humanized antibody" or a "humanized antibody fragment" is a specific type of chimeric antibody which includes an immunoglobulin amino acid sequence variant, or fragment thereof, which is capable of binding to a predetermined antigen and which, comprises one or more FRs having substantially the amino acid sequence of a human immunoglobulin and one or more CDRs having substantially the amino acid sequence of a non-human immunoglobulin. This non-human amino acid sequence often referred to as an "import" sequence is typically taken from an "import" antibody domain, particularly a variable domain. In general, a humanized antibody includes at least the CDRs or HVLs of a non-human antibody, inserted between the FRs of a human heavy or light chain variable domain. The present invention describes specific humanized anti-IL-23A antibodies which contain CDRs derived from the mouse monoclonal antibodies or humanized CDRs shown in Tables 1 and 2 inserted between the FRs of human germline sequence heavy and light chain variable domains. It will be understood that certain mouse FR residues may be important to the function of the humanized antibodies and therefore certain of the human germline sequence heavy and light chain variable domains residues are modified to be the same as those of the corresponding mouse sequence.

In another aspect, a humanized anti-IL-23A antibody comprises substantially all of at least one, and typically two, variable domains (such as contained, for example, in Fab, Fab', F(ab')2, Fabc, and Fv fragments) in which all, or substantially all, of the CDRs correspond to those of a non-human immunoglobulin, and specifically herein, all of the CDRs are mouse or humanized sequences as detailed in Tables 1 and 2 herein below and all, or substantially all, of the FRs are those of a human immunoglobulin consensus or germline sequence. In another aspect, a humanized anti- IL-23A antibody also includes at least a portion of an immunoglobulin Fc region, typically that of a human immunoglobulin. Ordinarily, the antibody will contain both the light chain as well as at least the variable domain of a heavy chain. The antibody also may include one or more of the C_{H1}, hinge, C_{H2}, C_{H3}, and/or C_{H4} regions of the heavy chain, as appropriate.

A humanized anti-IL-23A antibody can be selected from any class of immunoglobulins, including IgM, IgG, IgD, IgA and IgE, and any isotype, including IgG₁, IgG₂, IgG₃, IgG₄, IgA₁ and IgA₂. For example, the constant domain can be a complement fixing constant domain where it is desired that the humanized antibody exhibit cytotoxic activity, and the isotype is typically IgG₁. Where such cytotoxic activity is not desirable, the constant domain may be of another isotype, e.g., IgG₂. An alternative humanized anti-IL-23A antibody can comprise sequences from more than one immunoglobulin class or isotype, and selecting particular constant domains to optimize desired effector functions is within the ordinary skill in the art. In specific embodiments, the present invention provides antibodies that are IgG1 antibodies and more particularly, are IgG1 antibodies in which there is a knock-out of effector functions.

The FRs and CDRs, or HVLs, of a humanized anti-IL-23A antibody need not correspond precisely to the parental sequences. For example, one or more residues in the import CDR, or HVL, or the consensus or germline FR sequence may be altered (e.g., mutagenized) by substitution, insertion or deletion such that the resulting amino acid residue is no longer identical to the original residue in the corresponding position in either parental sequence but the antibody nevertheless retains the function of binding to IL-23A. Such alteration typically will not be extensive and will be conservative alterations. Usually, at least 75% of the humanized antibody residues will correspond to those of the parental consensus or germline FR and import CDR sequences, more often at least 90%, and most frequently greater than 95%, or greater than 98% or greater than 99%.

Immunoglobulin residues that affect the interface between heavy and light chain variable regions ("the V_{L}-V_{H} interface") are those that affect the proximity or orientation of the two chains with respect to one another. Certain residues that may be involved in interchain interactions include V_{L} residues 34, 36, 38, 44, 46, 87, 89, 91, 96, and 98 and V_{H} residues 35, 37, 39, 45, 47, 91, 93, 95, 100, and 103 (utilizing the numbering system set forth in Kabat et al., Sequences of Proteins of Immunological Interest (National Institutes of Health, Bethesda, Md., 1987)). U.S. Pat. No. 6,407,213 also discusses that residues such as V_{L} residues 43 and 85, and V_{H} residues 43 and 60 also may be involved in this interaction. While these residues are indicated for human IgG only, they are applicable across species. Important antibody residues that are reasonably expected to be involved in interchain interactions are selected for substitution into the consensus sequence.

The terms "consensus sequence" and "consensus antibody" refer to an amino acid sequence which comprises the most frequently occurring amino acid residue at each location in all immunoglobulins of any particular class, isotype, or subunit structure, e.g., a human immunoglobulin variable domain. The consensus sequence may be based on immunoglobulins of a particular species or of many species. A "consensus" sequence, structure, or antibody is understood to encompass a consensus human sequence as described in certain embodiments, and to refer to an amino acid sequence which comprises the most frequently occurring amino acid residues at each location in all human immunoglobulins of any particular class, isotype, or subunit structure. Thus, the consensus sequence contains an amino acid sequence having at each position an amino acid that is present in one or more known immunoglobulins, but which may not exactly duplicate the entire amino acid sequence of any single immunoglobulin. The variable region consensus sequence is not obtained from any naturally produced antibody or immunoglobulin. Kabat et al., 1991, Sequences of Proteins of Immunological Interest, 5th Ed. Public Health Service, National Institutes of Health, Bethesda, Md., and variants thereof. The FRs of heavy and light chain consensus sequences, and variants thereof, provide useful sequences for the preparation of humanized anti-IL-23p19 antibodies. See, for example, U.S. Pat. Nos. 6,037,454 and 6,054,297.

Human germline sequences are found naturally in the human population. A combination of those germline genes generates antibody diversity. Germline antibody sequences for the light chain of the antibody come from conserved human germline kappa or lambda v-genes and j-genes. Similarly the heavy chain sequences come from germline v-, d- and j-genes (LeFranc, M-P, and LeFranc, G, "The Immunoglobulin Facts Book" Academic Press, 2001).

As used herein, "variant", "anti- IL-23A variant", "humanized anti- IL-23A variant", or "variant humanized anti- IL-23A" each refers to a humanized anti-IL-23A antibody having at least a light chain variable murine CDR from any of the sequences as shown in Table 1 or a heavy chain murine CDR sequence derived from the murine monoclonal antibody as shown in Table 2. Variants include those having one or more amino acid changes in one or both light chain or heavy chain variable domains, provided that the amino acid change does not substantially impair binding of the antibody to IL-23A. Exemplary antibodies produced herein include those designated as Antibody A, Antibody B, Antibody C and Antibody D, and the various light chains and heavy chains of the same are shown in SEQ ID Nos:18 and 21, and SEQ ID Nos:19 and 20, respectively.

An "isolated" antibody is one that has been identified and separated and/or recovered from a component of its natural environment. Contaminant components of the antibody's natural environment are those materials that may interfere with diagnostic or therapeutic uses of the antibody, and can be enzymes, hormones, or other proteinaceous or nonproteinaceous solutes. In one aspect, the antibody will be purified to at least greater than 95% isolation by weight of antibody.

An isolated antibody includes an antibody in situ within recombinant cells in which it is produced, since at least one component of the antibody's natural environment will not be present. Ordinarily however, an isolated antibody will be prepared by at least one purification step in which the recombinant cellular material is removed.

The term "antibody performance" refers to factors that contribute to antibody recognition of antigen or the effectiveness of an antibody in vivo. Changes in the amino acid sequence of an antibody can affect antibody properties such as folding, and can influence physical factors such as initial rate of antibody binding to antigen (kₐ), dissociation constant of the antibody from antigen (k_{d}), affinity constant of the antibody for the antigen (Kd), conformation of the antibody, protein stability, and half-life of the antibody.

The term "epitope tagged" when used herein, refers to an anti-IL-23A antibody fused to an "epitope tag". An "epitope tag" is a polypeptide having a sufficient number of amino acids to provide an epitope for antibody production, yet is designed such that it does not interfere with the desired activity of the anti-IL-23A antibody. The epitope tag is usually sufficiently unique such that an antibody raised against the epitope tag does not substantially cross-react with other epitopes. Suitable tag polypeptides generally contain at least 6 amino acid residues and usually contain about 8 to 50 amino acid residues, or about 9 to 30 residues. Examples of epitope tags and the antibody that binds the epitope include the flu HA tag polypeptide and its antibody 12CA5 (Field et al., 1988 Mol. Cell. Biol. 8: 2159-2165; c-myc tag and 8F9, 3C7, 6E10, G4, B7 and 9E10 antibodies thereto (Evan et al., 1985, Mol. Cell. Biol. 5(12):3610-3616; and Herpes simplex virus glycoprotein D (gD) tag and its antibody (Paborsky et al. 1990, Protein Engineering 3(6): 547-553). In certain embodiments, the epitope tag is a "salvage receptor binding epitope". As used herein, the term "salvage receptor binding epitope" refers to an epitope of the Fc region of an IgG molecule (such as IgG₁, IgG₂, IgG₃, or IgG₄) that is responsible for increasing the in vivo serum half-life of the IgG molecule.

For diagnostic as well as therapeutic monitoring purposes, the antibodies of the invention also may be conjugated to a label, either a label alone or a label and an additional second agent (prodrug, chemotherapeutic agent and the like). A label, as distinguished from the other second agents refers to an agent that is a detectable compound or composition and it may be conjugated directly or indirectly to an antibody of the present invention. The label may itself be detectable (e.g., radioisotope labels or fluorescent labels) or, in the case of an enzymatic label, may catalyze chemical alteration of a substrate compound or composition that is detectable. Labeled anti-IL-23A antibody can be prepared and used in various applications including in vitro and in vivo diagnostics.

In various aspects of the present invention one or more domains of the antibodies will be recombinantly expressed. Such recombinant expression may employ one or more control sequences, i.e., polynucleotide sequences necessary for expression of an operably linked coding sequence in a particular host organism. The control sequences suitable for use in prokaryotic cells include, for example, promoter, operator, and ribosome binding site sequences. Eukaryotic control sequences include, but are not limited to, promoters, polyadenylation signals, and enhancers. These control sequences can be utilized for expression and production of anti-IL-23A antibody in prokaryotic and eukaryotic host cells.

A nucleic acid sequence is "operably linked" when it is placed into a functional relationship with another nucleic acid sequence. For example, a nucleic acid presequence or secretory leader is operably linked to a nucleic acid encoding a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers are optionally contiguous. Linking can be accomplished by ligation at convenient restriction sites. If such sites do not exist, synthetic oligonucleotide adaptors or linkers can be used.

As used herein, the expressions "cell", "cell line", and "cell culture" are used interchangeably and all such designations include the progeny thereof. Thus, "transformants" and "transformed cells" include the primary subject cell and cultures derived therefrom without regard for the number of transfers.

The term "mammal" for purposes of treatment refers to any animal classified as a mammal, including humans, domesticated and farm animals, and zoo, sports, or pet animals, such as dogs, horses, cats, cows, and the like. Preferably, the mammal is human.

A "disorder", as used herein, is any condition that would benefit from treatment with an anti-IL-23A antibody described herein. This includes chronic and acute disorders or diseases including those pathological conditions that predispose the mammal to the disorder in question.

As used herein, the term "IL-23-associated disorder" or "IL-23-associated disease" refers to a condition in which IL-23 activity contributes to the disease and typically where IL-23 is abnormally expressed. An IL-23-associated disorder includes diseases and disorders of the immune system, such as autoimmune disorders and inflammatory diseases. Such conditions include psoriasis, inflammatory bowel disease, for example ulcerative colitis or Crohn's disease, and spondyloarthritis, for example ankylosing spondylitis, non-radiographic axial spondyloarthritis, peripheral spondyloarthritis or psoriatic arthritis.

The term "intravenous infusion" refers to introduction of an agent into the vein of an animal or human patient over a period of time greater than approximately 15 minutes, generally between approximately 30 to 90 minutes.

The term "intravenous bolus" or "intravenous push" refers to drug administration into a vein of an animal or human such that the body receives the drug in approximately 15 minutes or less, generally 5 minutes or less.

The term "subcutaneous administration" refers to introduction of an agent under the skin of an animal or human patient, preferable within a pocket between the skin and underlying tissue, by relatively slow, sustained delivery from a drug receptacle. Pinching or drawing the skin up and away from underlying tissue may create the pocket.

The term "subcutaneous infusion" refers to introduction of a drug under the skin of an animal or human patient, preferably within a pocket between the skin and underlying tissue, by relatively slow, sustained delivery from a drug receptacle for a period of time including, but not limited to, 30 minutes or less, or 90 minutes or less. Optionally, the infusion may be made by subcutaneous implantation of a drug delivery pump implanted under the skin of the animal or human patient, wherein the pump delivers a predetermined amount of drug for a predetermined period of time, such as 30 minutes, 90 minutes, or a time period spanning the length of the treatment regimen.

The term "subcutaneous bolus" refers to drug administration beneath the skin of an animal or human patient, where bolus drug delivery is less than approximately 15 minutes; in another aspect, less than 5 minutes, and in still another aspect, less than 60 seconds. In yet even another aspect, administration is within a pocket between the skin and underlying tissue, where the pocket may be created by pinching or drawing the skin up and away from underlying tissue.

The term "therapeutically effective amount" is used to refer to an amount of an active agent that relieves or ameliorates one or more of the symptoms of the disorder being treated. In another aspect, the therapeutically effective amount refers to a target serum concentration that has been shown to be effective in, for example, slowing disease progression. Efficacy can be measured in conventional ways, depending on the condition to be treated.

The terms "treatment" and "therapy" and the like, as used herein, are meant to include therapeutic as well as prophylactic, or suppressive measures for a disease or disorder leading to any clinically desirable or beneficial effect, including but not limited to alleviation or relief of one or more symptoms, regression, slowing or cessation of progression of the disease or disorder. Thus, for example, the term treatment includes the administration of an agent prior to or following the onset of a symptom of a disease or disorder thereby preventing or removing one or more signs of the disease or disorder. As another example, the term includes the administration of an agent after clinical manifestation of the disease to combat the symptoms of the disease. Further, administration of an agent after onset and after clinical symptoms have developed where administration affects clinical parameters of the disease or disorder, such as the degree of tissue injury or the amount or extent of metastasis, whether or not the treatment leads to amelioration of the disease, comprises "treatment" or "therapy" as used herein. Moreover, as long as the compositions of the invention either alone or in combination with another therapeutic agent alleviate or ameliorate at least one symptom of a disorder being treated as compared to that symptom in the absence of use of the anti-IL-23A antibody composition, the result should be considered an effective treatment of the underlying disorder regardless of whether all the symptoms of the disorder are alleviated or not.

The term "package insert" is used to refer to instructions customarily included in commercial packages of therapeutic products, that contain information about the indications, usage, administration, contraindications and/or warnings concerning the use of such therapeutic products.

### Antibodies

The CDRs of selected antibodies used in the context of the present invention are shown in Table 1 and 2. The variable regions of selected antibodies used in the context of the present invention are shown in Table 3 and 4.

**Table 1: LIGHT CHAIN CDR sequences**

| | **L-CDR1** | **L-CDR2** | **L-CDR3** |
|---|---|---|---|
| **6B8** | **KASRDVAIAVA (SEQ ID NO:1)** | **WASTRHT (SEQ ID NO:2)** | **HQYSSYPFT (SEQ ID NO:3)** |

**Table 2: HEAVY CHAIN CDR sequences**

| | **H-CDR1** | **H-CDR2** | **H-CDR3** |
|---|---|---|---|
| **6B8** | **GNTFTDQTIH (SEQ ID NO:4)** | YIYPRDDSPKYNENFK G (SEQ ID NO:5) | **PDRSGYAWFIY (SEQ ID NO:6)** |
| **Hu_6B8-2** | **GYTFTDQTIH (SEQ ID NO:7)** | YIYPRDDSPKYNENFK G (SEQ ID NO:5) | **PDRSGYAWFIY (SEQ ID NO:6)** |
| **Hu_6B8-5** | **GFTFTDQTIH (SEQ ID NO:8)** | YIYPRDDSPKYNENFK G (SEQ ID NO:5) | **PDRSGYAWFIY (SEQ ID NO:6)** |
| **Hu_6B8-36/65** | **GGTFTDQTIH (SEQ ID NO:9)** | YIYPRDDSPKYNENFK G (SEQ ID NO:5) | **PDRSGYAWFIY (SEQ ID NO:6)** |

**Table 3: Humanized 6B8-VK Sequences**

| | |
|---|---|
| 6B8CVK-65 | DIQMTQSPSSLSASVGDRVTITCKASRDVAIAVAWYQQKPGKV PKLLLFWASTRHTGVPDRFSGSGSGTDFTLTISSLQPEDLADYY CHQYSSYPFTFGQGTKLEIK (SEQ ID NO:10) |
| 6B8CVK-66 | DIQMTQSPSSLSASVGDRVTITCKASRDVAIAVAWYQQKPGKV PKLLIYWASTRHTGVPSRFSGSGSRTDFTLTISSLQPEDVADYF CHQYSSYPFTFGSGTKLEIK (SEQ ID NO:11) |
| 6B8CVK-67 | DIQMTQSPSSLSASVGDRVTITCKASRDVAIAVAWYQQKPGKV PKLLLYWASTRHTGVPSRFSGSGSRTDFTLTISSLQPEDVATYY CHQYSSYPFTFGSGTKLEIK (SEQ ID NO:12) |
| 6B8CVK-78 | DIQMTQSPSSLSASVGDRVTITCKASRDVAIAVAWYQQKPGKV PKLLLFWASTRHTGVPDRFSGSGSRTDFTLTISSLQPEDLADYY CHQYSSYPFTFGSGTKLEIK (SEQ ID NO:13) |

**Table 4: Humanized 6B8-VH Sequence**

| | |
|---|---|
| 6B8CVH-02 | QVQLVQSGAEVKKPGSSVKVSCKASGYTFTDQTIHWMRQAPG QGLEWIGYIYPRDDSPKYNENFKGKVTITADKSTSTAYMELSS LRSEDTAVYYCAIPDRSGYAWFIYWGQGTLVTVSS (SEQ ID NO:14) |
| 6B8CVH-05 | QVQLVQSGAEVKKPGSSVKVSCKASGFTFTDQTIHWVRQAPG QGLEWMGYIYPRDDSPKYNENFKGKVTLTADKSTSTAYMELS SLRSEDTAVYYCAIPDRSGYAWFIYWGQGTLVTVSS (SEQ ID NO:15) |
| 6B8CVH-36 | QVQLVQSGAEVKKPGSSVKTSCKASGGTFTDQTIHWVRQRPG QGLEWMGYIYPRDDSPKYNENFKGRVTITADKSTSTAYMELS |
| | SLRSEDTAVYYCAIPDRSGYAWFIYWGQGTLVTVSS (SEQ ID NO:16) |
| 6B8CVH-65 | QVQLVQSGAEVKKPGSSVKVSCKASGGTFTDQTIHWVRQAPG QGLEWMGYIYPRDDSPKYNENFKGRVTLTADKSTSTAYMELS SLRSEDTAVYFCARPDRSGYAWFIYWGQGTLVTVSS (SEQ ID NO:17) |

Selected combination of humanized light chain and heavy chain variable regions derived from mouse antibody 6B8 resulted in Antibodies A, B, C and D:
Antibody A: 6B8-IgG1KO-2 with IgK-66 (heavy chain variable region 6B8CVH-02 and light chain variable region 6B8CVK-66);
Antibody B: 6B8-IgG1KO-5 with IgK-66 (heavy chain variable region 6B8CVH-05 and light chain variable region 6B8CVK-66);
Antibody C: 6B8-IgG1KO-2 with IgK-65 (heavy chain variable region 6B8CVH-02 and light chain variable region 6B8CVK-65);
Antibody D: 6B8-IgG1KO-5 with IgK-65 (heavy chain variable region 6B8CVH-05 and light chain variable region 6B8CVK-65).

Antibodies A, B, C and D have the heavy and light chain sequences shown in Table 5.

**Table 5: Heavy and Light Chain DNA and Amino Acid Sequences for Antibodies A, B, C, and D**

| | | |
|---|---|---|
| **Antibody A** | IgK light Chain #66 | DIQMTQSPSSLSASVGDRVTITCKASRDVAIAVAW YQQKPGKVPKLLIYWASTRHTGVPSRFSGSGSRT DFTLTISSLQPEDVADYFCHQYSSYPFTFGSGTKL EIKRTVAAPSVFIFPPSDEQLKSGTASVVCLLNNFY |
| | | PREAKVQWKVDNALQSGNSQESVTEQDSKDSTY SLSSTLTLSKADYEKHKVYACEVTHQGLSSPVTK SFNRGEC (SEQ ID NO:18) |
| | IgG1 KO Heavy Chain #2 | QVQLVQSGAEVKKPGSSVKVSCKASGYTFTDQTI HWMRQAPGQGLEWIGYIYPRDDSPKYNENFKGK VTITADKSTSTAYMELSSLRSEDTAVYYCAIPDRS GYAWFIYWGQGTLVTVSSASTKGPSVFPLAPSSK STSGGTAALGCLVKDYFPEPVTVSWNSGALTSGV HTFPAVLQSSGLYSLSSVVTVPSSSLGTQTYICNV NHKPSNTKVDKRVEPKSCDKTHTCPPCPAPEAAG GPSVFLFPPKPKDTLMISRTPEVTCVVVDVSHEDP EVKFNWYVDGVEVHNAKTKPREEQYNSTYRVVS VLTVLHQDWLNGKEYKCKVSNKALPAPIEKTISK AKGQPREPQVYTLPPSREEMTKNQVSLTCLVKGF YPSDIAVEWESNGQPENNYKTTPPVLDSDGSFFLY SKLTVDKSRWQQGNVFSCSVMHEALHNHYTQKS LSLSPG (SEQ ID NO:19) |
| | | |
| **Antibody B** | IgK light Chain #66 | (SEQ ID NO:18) |
| | IgG1KO Heavy Chain #5 | QVQLVQSGAEVKKPGSSVKVSCKASGFTFTDQTI HWVRQAPGQGLEWMGYIYPRDDSPKYNENFKG KVTLTADKSTSTAYMELSSLRSEDTAVYYCAIPD RSGYAWFIYWGQGTLVTVSSASTKGPSVFPLAPS SKSTSGGTAALGCLVKDYFPEPVTVSWNSGALTS GVHTFPAVLQSSGLYSLSSVVTVPSSSLGTQTYIC NVNHKPSNTKVDKRVEPKSCDKTHTCPPCPAPEA |
| | | AGGPSVFLFPPKPKDTLMISRTPEVTCVVVDVSHE DPEVKFNWYVDGVEVHNAKTKPREEQYNSTYRV VSVLTVLHQDWLNGKEYKCKVSNKALPAPIEKTI SKAKGQPREPQVYTLPPSREEMTKNQVSLTCLVK GFYPSDIAVEWESNGQPENNYKTTPPVLDSDGSFF LYSKLTVDKSRWQQGNVFSCSVMHEALHNHYTQ KSLSLSPG (SEQ ID NO:20) |
| | | |
| **Antibody C** | IgK light Chain #65 | DIQMTQSPSSLSASVGDRVTITCKASRDVAIAVAW YQQKPGKVPKLLLFWASTRHTGVPDRFSGSGSGT DFTLTISSLQPEDLADYYCHQYSSYPFTFGQGTKL EIKRTVAAPSVFIFPPSDEQLKSGTASVVCLLNNFY PREAKVQWKVDNALQSGNSQESVTEQDSKDSTY SLSSTLTLSKADYEKHKVYACEVTHQGLSSPVTK SFNRGEC (SEQ ID NO:21) |
| | IgG1KO Heavy Chain #2 | (SEQ ID NO:19) |
| | | |
| **Antibody D** | IgK light Chain #65 | (SEQ ID NO:21) |
| | IgG1KO Heavy Chain #5 | (SEQ ID NO:20) |

Light chains and heavy chain variable regions of Antibodies A, B, C, and D are underlined in Table 5 above.

In one embodiment, an anti-IL-23A antibody comprises the light chain sequence of SEQ ID NO:18 and the heavy chain sequence of SEQ ID NO:19. In one embodiment, an anti-IL-23A antibody comprises the light chain sequence of SEQ ID NO:18 and the heavy chain sequence of SEQ ID NO:20. In one embodiment, an anti-IL-23A antibody comprises the light chain sequence of SEQ ID NO:21 and the heavy chain sequence of SEQ ID NO:19. In one embodiment, an anti-IL-23A antibody comprises the light chain sequence of SEQ ID NO:21 and the heavy chain sequence of SEQ ID NO:20.

In one embodiment, an anti-IL-23A antibody consists of the light chain sequence of SEQ ID NO:18 and the heavy chain sequence of SEQ ID NO:19. In one embodiment, an anti-IL-23A antibody consists of the light chain sequence of SEQ ID NO:18 and the heavy chain sequence of SEQ ID NO:20. In one embodiment, an anti-IL-23A antibody consists of the light chain sequence of SEQ ID NO:21 and the heavy chain sequence of SEQ ID NO:19. In one embodiment, an anti-IL-23A antibody consists of the light chain sequence of SEQ ID NO:21 and the heavy chain sequence of SEQ ID NO:20.

In a further embodiment, an anti-IL-23A antibody binds to human IL-23A at an epitope consisting of amino acid residues 108 to 126 and amino acid residues 137 to 151 of SEQ ID NO: 22.

In a further embodiment, an anti-IL-23A antibody competitively binds to human IL-23A with an antibody of the present invention, for example Antibody A, Antibody B, Antibody C or Antibody D described herein. The ability of an antibody to competitively bind to IL-23A can be measured using competitive binding assays known in the art.

In some embodiments, an anti-IL-23A antibody comprises light chain variable region sequences having the amino acid sequence set forth in of SEQ ID NO:10, 11, 12 or 13. In some embodiments, an anti-IL-23A antibody comprises heavy chain variable region sequences having the amino acid sequence set forth in of SEQ ID NO:14, 15, 16 or 17 (see Tables 3 and 4 above). The CDR sequences of these antibodies are shown in Tables 1 and 2. For example, anti-IL-23A antibodies are monoclonal antibodies with the combinations of light chain variable and heavy chain variable regions of SEQ ID NO: 11/14, 11/15, 10/14 or 10/15. Such variable regions can be combined with human constant regions.

### Polynucleotides, Vectors, Host Cells, and Recombinant Methods

Other embodiments encompass isolated polynucleotides that comprise a sequence encoding an anti-IL-23A antibody, vectors, and host cells comprising the polynucleotides, and recombinant techniques for production of the humanized antibody. The isolated polynucleotides can encode any desired form of the anti-IL-23A antibody including, for example, full length monoclonal antibodies, Fab, Fab', F(ab')₂, and Fv fragments.

The polynucleotide(s) that comprise a sequence encoding an anti-IL-23A antibody can be fused to one or more regulatory or control sequence, as known in the art, and can be contained in suitable expression vectors or host cell as known in the art. Each of the polynucleotide molecules encoding the heavy or light chain variable domains can be independently fused to a polynucleotide sequence encoding a constant domain, such as a human constant domain, enabling the production of intact antibodies. Alternatively, polynucleotides, or portions thereof, can be fused together, providing a template for production of a single chain antibody.

For recombinant production, a polynucleotide encoding the antibody is inserted into a replicable vector for cloning (amplification of the DNA) or for expression. Many suitable vectors for expressing the recombinant antibody are available. The vector components generally include, but are not limited to, one or more of the following: a signal sequence, an origin of replication, one or more marker genes, an enhancer element, a promoter, and a transcription termination sequence.

The anti-IL-23A antibodies can also be produced as fusion polypeptides, in which the antibody is fused with a heterologous polypeptide, such as a signal sequence or other polypeptide having a specific cleavage site at the amino terminus of the mature protein or polypeptide. The heterologous signal sequence selected is typically one that is recognized and processed (i.e., cleaved by a signal peptidase) by the host cell. For prokaryotic host cells that do not recognize and process the anti-IL-23A antibody signal sequence, the signal sequence can be substituted by a prokaryotic signal sequence. The signal sequence can be, for example, alkaline phosphatase, penicillinase, lipoprotein, heat-stable enterotoxin II leaders, and the like. For yeast secretion, the native signal sequence can be substituted, for example, with a leader sequence obtained from yeast invertase alpha-factor (including Saccharomyces and Kluyveromyces α-factor leaders), acid phosphatase, C. albicans glucoamylase, or the signal described in WO90/13646. In mammalian cells, mammalian signal sequences as well as viral secretory leaders, for example, the herpes simplex gD signal, can be used. The DNA for such precursor region is ligated in reading frame to DNA encoding the anti-IL-23A antibody.

Expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2-υ plasmid origin is suitable for yeast, and various viral origins (SV40, polyoma, adenovirus, VSV, and BPV) are useful for cloning vectors in mammalian cells. Generally, the origin of replication component is not needed for mammalian expression vectors (the SV40 origin may typically be used only because it contains the early promoter).

Expression and cloning vectors may contain a gene that encodes a selectable marker to facilitate identification of expression. Typical selectable marker genes encode proteins that confer resistance to antibiotics or other toxins, e.g., ampicillin, neomycin, methotrexate, or tetracycline or alternatively, are complement auxotrophic deficiencies, or in other alternatives supply specific nutrients that are not present in complex media, e.g., the gene encoding D-alanine racemase for Bacilli.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid, and hygromycin. Common selectable markers for mammalian cells are those that enable the identification of cells competent to take up a nucleic acid encoding an anti-IL-23A antibody, such as DHFR (dihydrofolate reductase), thymidine kinase, metallothionein-I and -II (such as primate metallothionein genes), adenosine deaminase, ornithine decarboxylase, and the like. Cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity (e.g., DG44).

Alternatively, host cells (particularly wild-type hosts that contain endogenous DHFR) transformed or co-transformed with DNA sequences encoding an anti-IL-23A antibody, wild-type DHFR protein, and another selectable marker such as aminoglycoside 3'-phosphotransferase (APH), can be selected by cell growth in medium containing a selection agent for the selectable marker such as an aminoglycosidic antibiotic, e.g., kanamycin, neomycin, or G418. See, e.g., U.S. Pat. No. 4,965,199.

Where the recombinant production is performed in a yeast cell as a host cell, the TRP1 gene present in the yeast plasmid YRp7 (Stinchcomb et al., 1979, Nature 282: 39) can be used as a selectable marker. The TRP1 gene provides a selection marker for a mutant strain of yeast lacking the ability to grow in tryptophan, for example, ATCC No. 44076 or PEP4-1 (Jones, 1977, Genetics 85:12). The presence of the trp1 lesion in the yeast host cell genome then provides an effective environment for detecting transformation by growth in the absence of tryptophan. Similarly, Leu2p-deficient yeast strains such as ATCC 20,622 and 38,626 are complemented by known plasmids bearing the LEU2 gene.

In addition, vectors derived from the 1.6 µm circular plasmid pKD1 can be used for transformation of Kluyveromyces yeasts. Alternatively, an expression system for large-scale production of recombinant calf chymosin was reported for K. lactis (Van den Berg, 1990, Bio/Technology 8:135). Stable multi-copy expression vectors for secretion of mature recombinant human serum albumin by industrial strains of Kluyveromyces have also been disclosed (Fleer et al., 1991, Bio/Technology 9:968-975).

Expression and cloning vectors usually contain a promoter that is recognized by the host organism and is operably linked to the nucleic acid molecule encoding an anti-IL-23p19 antibody or polypeptide chain thereof. Promoters suitable for use with prokaryotic hosts include phoA promoter, β-lactamase and lactose promoter systems, alkaline phosphatase, tryptophan (trp) promoter system, and hybrid promoters such as the tac promoter. Other known bacterial promoters are also suitable. Promoters for use in bacterial systems also will contain a Shine-Dalgarno (S.D.) sequence operably linked to the DNA encoding the anti-IL-23A antibody.

Many eukaryotic promoter sequences are known. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Inducible promoters have the additional advantage of transcription controlled by growth conditions. These include yeast promoter regions for alcohol dehydrogenase 2, isocytochrome C, acid phosphatase, derivative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Anti-IL-23A antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, or from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., 1982, Nature 297:598-601, disclosing expression of human p-interferon cDNA in mouse cells under the control of a thymidine kinase promoter from herpes simplex virus. Alternatively, the Rous sarcoma virus long terminal repeat can be used as the promoter.

Another useful element that can be used in a recombinant expression vector is an enhancer sequence, which is used to increase the transcription of a DNA encoding an anti-IL-23A antibody by higher eukaryotes. Many enhancer sequences are now known from mammalian genes (e.g., globin, elastase, albumin, α-fetoprotein, and insulin). Typically, however, an enhancer from a eukaryotic cell virus is used. Examples include the SV40 enhancer on the late side of the replication origin (bp 100-270), the cytomegalovirus early promoter enhancer, the polyoma enhancer on the late side of the replication origin, and adenovirus enhancers. See also Yaniv, 1982, Nature 297:17-18 for a description of enhancing elements for activation of eukaryotic promoters. The enhancer may be spliced into the vector at a position 5' or 3' to the anti-IL-23A antibody-encoding sequence, but is preferably located at a site 5' from the promoter.

Expression vectors used in eukaryotic host cells (yeast, fungi, insect, plant, animal, human, or nucleated cells from other multicellular organisms) can also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding anti-IL-23A antibody. One useful transcription termination component is the bovine growth hormone polyadenylation region. See WO94/11026 and the expression vector disclosed therein. In some embodiments, humanized anti-IL-23p19 antibodies can be expressed using the CHEF system. (See, e.g., U.S. Pat. No. 5,888,809; the disclosure of which is incorporated by reference herein.)

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, e.g., E. coli, Enterobacter, Erwinia, Klebsiella, Proteus, Salmonella, e.g., Salmonella typhimurium, Serratia, e.g., Serratia marcescans, and Shigella, as well as Bacilli such as B. subtilis and B. licheniformis (e.g., B. licheniformis 41 P disclosed in DD 266,710 published Apr. 12, 1989), Pseudomonas such as P. aeruginosa, and Streptomyces. One preferred E. coli cloning host is E. coli 294 (ATCC 31,446), although other strains such as E. coli B, E. coli X1776 (ATCC 31,537), and E. coli W3110 (ATCC 27,325) are suitable. These examples are illustrative rather than limiting.

In addition to prokaryotes, eukaryotic microbes such as filamentous fungi or yeast are suitable cloning or expression hosts for anti-IL-23A antibody-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, e.g., K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastors (EP 183,070); Candida; Trichoderma reesia (EP 244,234); Neurospora crassa; Schwanniomyces such as Schwanniomyces occidentalis; and filamentous fungi such as, e.g., Neurospora, Penicillium, Tolypocladium, and Aspergillus hosts such as A. nidulans and A. niger.

Suitable host cells for the expression of glycosylated anti-IL-23A antibody are derived from multicellular organisms. Examples of invertebrate cells include plant and insect cells, including, e.g., numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruit fly), and Bombyx mori (silk worm). A variety of viral strains for transfection are publicly available, e.g., the L-1 variant of Autographa californica NPV and the Bm-5 strain of Bombyx mori NPV, and such viruses may be used, particularly for transfection of Spodoptera frugiperda cells.

Plant cell cultures of cotton, corn, potato, soybean, petunia, tomato, and tobacco can also be utilized as hosts.

In another aspect, expression of anti-IL-23A antibodies is carried out in vertebrate cells. The propagation of vertebrate cells in culture (tissue culture) has become routine procedure and techniques are widely available. Examples of useful mammalian host cell lines are monkey kidney CV1 line transformed by SV40 (COS-7, ATCC CRL 1651), human embryonic kidney line (293 or 293 cells subcloned for growth in suspension culture, (Graham et al., 1977, J. Gen Virol. 36: 59), baby hamster kidney cells (BHK, ATCC CCL 10), Chinese hamster ovary cells/- DHFR1 (CHO, Urlaub et al., 1980, Proc. Natl. Acad. Sci. USA 77: 4216; e.g., DG44), mouse sertoli cells (TM4, Mather, 1980, Biol. Reprod. 23:243-251), monkey kidney cells (CV1 ATCC CCL 70), African green monkey kidney cells (VERO-76, ATCC CRL-1587), human cervical carcinoma cells (HELA, ATCC CCL 2), canine kidney cells (MDCK, ATCC CCL 34), buffalo rat liver cells (BRL 3A, ATCC CRL 1442), human lung cells (W138, ATCC CCL 75), human liver cells (Hep G2, HB 8065), mouse mammary tumor (MMT 060562, ATCC CCL51), TR1 cells (Mather et al., 1982, Annals N.Y. Acad. Sci. 383: 44-68), MRC 5 cells, FS4 cells, and human hepatoma line (Hep G2).

Host cells are transformed with the above-described expression or cloning vectors for anti-IL-23A antibody production and cultured in conventional nutrient media modified as appropriate for inducing promoters, selecting transformants, or amplifying the genes encoding the desired sequences.

The host cells used to produce an anti-IL-23A antibody described herein may be cultured in a variety of media. Commercially available media such as Ham's F10 (Sigma-Aldrich Co., St. Louis, Mo.), Minimal Essential Medium ((MEM), (Sigma-Aldrich Co.), RPMI-1640 (Sigma-Aldrich Co.), and Dulbecco's Modified Eagle's Medium ((DMEM), Sigma-Aldrich Co.) are suitable for culturing the host cells. In addition, any of the media described in one or more of Ham et al., 1979, Meth. Enz. 58: 44, Barnes et al., 1980, Anal. Biochem. 102: 255, U.S. Pat. No. 4,767,704, U.S. Pat. No. 4,657,866, U.S. Pat. No. 4,927,762, U.S. Pat. No. 4,560,655, U.S. Pat. No. 5,122,469, WO 90/103430, and WO 87/00195 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as gentamicin), trace elements (defined as inorganic compounds usually present at final concentrations in the micromolar range), and glucose or an equivalent energy source. Other supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the antibody can be produced intracellularly, in the periplasmic space, or directly secreted into the medium. If the antibody is produced intracellularly, the cells may be disrupted to release protein as a first step. Particulate debris, either host cells or lysed fragments, can be removed, for example, by centrifugation or ultrafiltration. Carter et al., 1992, Bio/Technology 10:163-167 describes a procedure for isolating antibodies that are secreted to the periplasmic space of E. coli. Briefly, cell paste is thawed in the presence of sodium acetate (pH 3.5), EDTA, and phenylmethylsulfonylfluoride (PMSF) over about 30 minutes. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants. A variety of methods can be used to isolate the antibody from the host cell.

The antibody composition prepared from the cells can be purified using, for example, hydroxylapatite chromatography, gel electrophoresis, dialysis, and affinity chromatography, with affinity chromatography being a typical purification technique. The suitability of protein A as an affinity ligand depends on the species and isotype of any immunoglobulin Fc domain that is present in the antibody. Protein A can be used to purify antibodies that are based on human gamma1, gamma2, or gamma4 heavy chains (see, e.g., Lindmark et al., 1983 J. Immunol. Meth. 62:1-13). Protein G is recommended for all mouse isotypes and for human gamma3 (see, e.g., Guss et al., 1986 EMBO J. 5:1567-1575). A matrix to which an affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C_{H3} domain, the Bakerbond ABX™ resin (J. T. Baker, Phillipsburg, N.J.) is useful for purification. Other techniques for protein purification such as fractionation on an ion-exchange column, ethanol precipitation, reverse phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, typically performed at low salt concentrations (e.g., from about 0-0.25M salt).

### Therapeutic Uses

In another embodiment, an anti-IL-23A antibody disclosed herein is useful in the treatment of various disorders associated with the expression of IL-23p19 as described herein. In one aspect, a method for treating an IL-23 associated disorder comprises administering a therapeutically effective amount of an anti-IL-23A antibody to a subject in need thereof.

The anti-IL-23A antibody is administered by any suitable means, including parenteral, subcutaneous, intraperitoneal, intrapulmonary, and intranasal, and, if desired for local immunosuppressive treatment, intralesional administration (including perfusing or otherwise contacting the graft with the antibody before transplantation). The anti-IL-23A antibody or agent can be administered, for example, as an infusion or as a bolus. Parenteral infusions include intramuscular, intravenous, intraarterial, intraperitoneal, or subcutaneous administration. In addition, the anti-IL-23A antibody is suitably administered by pulse infusion, particularly with declining doses of the antibody. In one aspect, the dosing is given by injections, most preferably intravenous or subcutaneous injections, depending in part on whether the administration is brief or chronic. In one aspect, the dosing of the anti-IL-23 antibody is given by subcutaneous injections.

For the prevention or treatment of disease, the appropriate dosage of antibody will depend on a variety of factors such as the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

The term "suppression" is used herein in the same context as "amelioration" and "alleviation" to mean a lessening of one or more characteristics of the disease.

The antibody is formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the antibody to be administered will be governed by such considerations.

The antibody may optionally be formulated with one or more agents currently used to prevent or treat the disorder in question. The effective amount of such other agents depends on the amount of anti-IL-23A antibody present in the formulation, the type of disorder or treatment, and other factors discussed above.

### IL-23-Associated Disorders

The anti-IL-23p19 antibodies or agents are useful for treating or preventing an immunological disorder characterized by abnormal expression of IL-23, e.g., by inappropriate activation of immune cells (e.g., lymphocytes or dendritic cells). Such abnormal expression of IL-23 can be due to, for example, increased IL-23 protein levels.

Immunological diseases that are characterized by inappropriate activation of immune cells and that can be treated or prevented by the methods described herein can be classified, for example, by the type(s) of hypersensitivity reaction(s) that underlie the disorder. These reactions are typically classified into four types: anaphylactic reactions, cytotoxic (cytolytic) reactions, immune complex reactions, or cell-mediated immunity (CMI) reactions (also referred to as delayed-type hypersensitivity (DTH) reactions). (See, e.g., Fundamental Immunology (William E. Paul ed., Raven Press, N.Y., 3rd ed. 1993).) Immunological diseases include inflammatory diseases and autoimmune diseases.

Examples of immunological diseases include the following: psoriasis, inflammatory bowel disease, for example ulcerative colitis or Crohn's disease, and spondyloarthritis, for example ankylosing spondylitis, non-radiographic axial spondyloarthritis, peripheral spondyloarthritis or psoriatic arthritis.

In one aspect, in the context of the present invention, the immunological disease is psoriasis. Psoriasis is a chronic inflammatory disease of the skin characterized by dysfunctional keratinocyte differentiation and hyperproliferation and marked accumulation of inflammatory T cells and dendritic cells. For example, the immunological disease includes plaque psoriasis, for example chronic plaque psoriasis, for example moderate to severe chronic plaque psoriasis, for example in patients who are candidates for systemic therapy or phototherapy. In one aspect, the immunological disease is moderate to severe plaque psoriasis and the patient is a patient who failed to respond to, or who has a contraindication to, or is intolerant to other systemic therapy including ciclosporin, methotrexate, psoralen or ultraviolet-A light (PUVA).

For example, the immunological disease includes palmar pustular psoriasis, guttate psoriasis, inverse psoriasis, pustular psoriasis or erythodermic psoriasis (also known as psoriatic erythroderma). For example, the immunological disease includes generalized pustular psoriasis (GPP).

For psoriasis, disease severity can be characterized by body surface area (BSA) involvement with <5% being considered mild, 5-10% moderate and > 10% severe. In some cases, disease status is measured using the Psoriasis Area and Severity Index (PASI), a composite measure of erythema, induration, desquamation and BSA affected by psoriasis with a range of scores from 0 to 72. In one aspect, the percentage of patients reaching PASI₇₅ (PASI 75), a 75% reduction in score from baseline at a certain time (for example 12 or 16 weeks) is used as a primary endpoint in psoriasis treatment, for example in psoriasis treatment trials. In one aspect, the percentage of patients reaching a PASI₉₀ (PASI 90), a 90% reduction in score from baseline at a certain time (for example at week 12 or 16) is used as primary endpoint in psoriasis treatment, for example in psoriasis treatment trials.

In one aspect, the percentage of patients reaching a PASI₉₀ at week 12 pursuant to a method according to the present invention, for example using Antibody A, is at least 60%, at least 65% or at least 70%.

In one aspect, the percentage of patients reaching a sPGA score of clear (score of 0) or almost clear (score of 1) at a certain time (for example at week 12 or 16) is used as primary endpoint in psoriasis treatment, for example in psoriasis treatment trials.

In one aspect, a patient treated within the context of the instant invention is a patient with stable moderate to severe chronic plaque-type psoriasis with or without psoriatic arthritis involving 10% or more body surface area, with disease severity PASI equal or greater than 12 and/or sPGA score of moderate and above (score of at least 3). In one aspect, a patient treated within the context of the instant invention is a patient with a psoriasis disease duration of at least 6 months prior to initiating the treatment.

In one aspect, in the context of the present invention, the immunological disease is psoriatic arthritis. For psoriatic arthritis, a patient has psoriatic skin lesions or a history of psoriasis in combination with nail dystrophy, inflammation of the digits (dactylitis) and a characteristic radiographic appearance (ill-defined ossification near joint margins) with a negative serum test for rheumatoid factor. Efficacy of treatment of psoriatic arthritis in clinical trials is frequently gauged using the percentage of patients achieving the American College of Rheumatology (ACR) composite measure of efficacy, as has been used prominently in trials in rheumatoid arthritis. The ACR20 indicates achievement of at least a 20% improvement in the number of swollen and tender joint counts as well as 20% improvement in 3 of 5 ACR core set measures when comparing baseline to some point in time after initiation of treatment e.g., at 24 weeks.

In one aspect, the percentage of ACR20 responders (20% improvement in American College of Rheumatology response criteria), for example at 24 weeks, is used as primary endpoint in psoriatic arthritis treatment, for example in psoriatic arthritis treatment trials.

In one aspect, immunological disease is psoriatic arthritis, for example active psoriatic arthritis, and the anti-IL-23A antibody is used alone or in combination with one or more non-biologic DMARDs (Disease-Modifying Antirheumatic Drug), for example to reduce signs and symptoms. In one aspect, the anti-IL-23A antibody is used or indicated to inhibit the progression of structural damage, and/or improve physical function.

In one aspect, the anti-IL-23A antibody is used alone or in combination with methotrexate (MTX) for the treatment of psoriatic arthritis, for example active psoriatic arthritis, for example when the response to previous non-biological DMARD therapy has been inadequate. In one aspect, the anti-IL-23A antibody is used to reduce the rate of progression of peripheral joint damage as measured by X-ray and/or to improve physical function.

In one aspect, in the context of the present invention, the immunological disease is axial (spinal) spondyloarthritis (ax-SpA), including ankylosing spondylitis (AS, also called radiographic ax-SpA) and non-radiographic ax-SpA.

In one aspect, in the context of the present invention, the immunological disease is Ankylosing Spondylitis. Ankylosing Spondylitis (AS) is an inflammatory disease involving primarily the axial skeleton and sacroiliac joints. Other musculoskeletal manifestations of the disease include peripheral arthritis and enthesitis. Extra-articular disease includes anterior uveitis, osteoporosis, cardiac disease with primarily valvular involvement, renal disease, lung disease, gastrointestinal disease, and skin disease. AS has a male predominance with a male-to-female ratio of 3:1. The peak age of onset is typically in the second or third decade of life. Estimates of the prevalence of AS worldwide range from approximately 0.5% to 0.07%. Patients with AS are likely to lose their physical function and ability to work, which is likely to have a significant impact on the quality of life unless an appropriate treatment is administered to the patients.

For ankylosing spondylitis, a patient has inflammatory back pain, asymmetrical peripheral arthritis, inflammation of the tendons (enthesitis) and extra-articular features, such as uveitis, and a strong genetic association with human leucocyte antigen B27. In one aspect, the percentage Ankylosing Spondylitis Disease Activity Score 40 (ASDAS 40) responders, for example at 24 weeks is used as primary endpoint in ankylosing spondylitis treatment, for example in ankylosing spondylitis treatment trials.

In one aspect, in the context of the present invention, the immunological disease is non-radiographic axial spondyloarthritis (ax-SpA). Non-radiographic axial spondyloarthritis, a more recently defined entity than AS, is considered to represent an earlier manifestation of the same pathologic processes as AS, although it is increasingly recognized that some patients (particularly females) may not progress to radiographic disease, and may therefore be considered to have a distinct subtype of this disease.

### Pharmaceutical Compositions and Administration Thereof

A composition comprising an anti-IL-23A antibody can be administered to a subject having or at risk of having an immunological disorder. The term "subject" as used herein means any mammalian patient to which an anti-IL-23A antibody can be administered, including, e.g., humans and non-human mammals, such as primates, rodents, and dogs. Subjects specifically intended for treatment using the methods described herein include humans. The antibodies can be administered either alone or in combination with other compositions in the prevention or treatment of the immunological disorder.

Anti-IL-23A antibodies for use in such pharmaceutical compositions are described herein, for example Antibody A, Antibody B, Antibody C or Antibody D.

Various delivery systems are known and can be used to administer the anti-IL-23A antibody. Methods of introduction include but are not limited to intradermal, intramuscular, intraperitoneal, intravenous, subcutaneous, intranasal, epidural, and oral routes. The anti-IL-23A antibody can be administered, for example by infusion, bolus or injection, and can be administered together with other biologically active agents such as chemotherapeutic agents. Administration can be systemic or local. In one embodiment, the administration is by subcutaneous injection. Formulations for such injections may be prepared in for example prefilled syringes that may be administered once every other week.

In specific embodiments, the anti-IL-23A antibody is administered by injection, by means of a catheter, by means of a suppository, or by means of an implant, the implant being of a porous, non-porous, or gelatinous material, including a membrane, such as a sialastic membrane, or a fiber. Typically, when administering the composition, materials to which the anti-IL-23A antibody or agent does not absorb are used.

In other embodiments, the anti-IL-23A antibody is delivered in a controlled release system. In one embodiment, a pump may be used (see, e.g., Langer, 1990, Science 249:1527-1533; Sefton, 1989, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used. (See, e.g., Medical Applications of Controlled Release (Langer and Wise eds., CRC Press, Boca Raton, Fla., 1974); Controlled Drug Bioavailability, Drug Product Design and Performance (Smolen and Ball eds., Wiley, New York, 1984); Ranger and Peppas, 1983, Macromol. Sci. Rev. Macromol. Chem. 23:61. See also Levy et al., 1985, Science 228:190; During et al., 1989, Ann. Neurol. 25:351; Howard et al., 1989, J. Neurosurg. 71:105.) Other controlled release systems are discussed, for example, in Langer, supra.

An anti-IL-23p19 antibody is typically administered as pharmaceutical compositions comprising a therapeutically effective amount of the antibody and one or more pharmaceutically compatible ingredients.

In typical embodiments, the pharmaceutical composition is formulated in accordance with routine procedures as a pharmaceutical composition adapted for intravenous or subcutaneous administration to human beings. Typically, compositions for administration by injection are solutions in sterile isotonic aqueous buffer. Where necessary, the pharmaceutical can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the pharmaceutical is to be administered by infusion, it can be dispensed with an infusion bottle containing sterile pharmaceutical grade water or saline. Where the pharmaceutical is administered by injection, an ampoule of sterile water for injection or saline can be provided so that the ingredients can be mixed prior to administration.

Further, the pharmaceutical composition can be provided as a pharmaceutical kit comprising (a) a container containing an anti-IL-23A antibody in lyophilized form and (b) a second container containing a pharmaceutically acceptable diluent (e.g., sterile water) for injection. The pharmaceutically acceptable diluent can be used for reconstitution or dilution of the lyophilized anti-IL-23A antibody. Optionally associated with such container(s) can be a notice in the form prescribed by a governmental agency regulating the manufacture, use or sale of pharmaceuticals or biological products, which notice reflects approval by the agency of manufacture, use or sale for human administration.

Examples of pharmaceutical compositions used in the context of the present invention are disclosed in Example 4 hereinbelow.

The invention is further described in the following examples, which are not intended to limit the scope of the invention.

### Examples

### Example 1: Clinical Studies

The results shown in Examples 1a, 1b and 1c are derived from the same clinical study.

### Example 1a: Study

This study assessed the efficacy and safety of Antibody A vs ustekinumab in patients with moderate-to-severe plaque psoriasis.

166 patients were randomly assigned to one of three dose regimens of Antibody A (18 mg single injection; 90 or 180 mg at weeks 0 and 4) or ustekinumab (45 or 90 mg). PASI (Psoriasis Area and Severity Index), sPGA (Static Physician Global Assessment) and safety parameters were assessed. Primary endpoint was achievement of PASI 90 (achievement of ≥ 90% reduction from baseline PASI score) at week 12, with primary analysis comparing the two highest doses of Antibody A (pooled) with ustekinumab.

The primary analysis showed Antibody A to be superior to ustekinumab (PASI 90 response rate of 77.1% for Antibody A vs 40% for ustekinumab [p<0.0001]). sPGA scores of 0 (clear) or 1 (almost clear) were achieved by 90.4% of Antibody A patients compared with 67.5% of ustekinumab patients. Complete clearing of lesions (PASI 100) was achieved in 45.8% of Antibody A patients and 17.5% of ustekinumab patients. AEs were similar across treatment groups with no drug-related severe or serious AEs (adverse events).

Selective blockade of IL-23p19 with Antibody A is associated with superior clinical response to ustekinumab in patients with moderate-to-severe plaque psoriasis.

### Example 1b: Study

**Objectives:** The efficacy and safety of the selective IL-23p19 antagonist monoclonal antibody Antibody A were assessed and compared with ustekinumab in patients with moderate-to-severe plaque psoriasis (including those with and without concurrent psoriatic arthritis (PsA)).

**Methods:** In this Phase 2 study, 166 patients were randomly assigned (1:1:1:1 ratio) to receive subcutaneous injections of one of three dose regimens of Antibody A (18 mg single dose at week 0; 90 or 180 mg at weeks 0, 4 and 16) or ustekinumab (45 or 90 mg at weeks 0, 4 and 16). Skin lesions were assessed using the Psoriasis Area and Severity Index (PASI) with the primary endpoint of PASI 90 (90% improvement from baseline) at 12 weeks. In patients who had concurrent PsA (either diagnosed by rheumatologist or suspected), the pain was assessed using the Visual Analogue Scale (pain-VAS) at baseline and at weeks 4, 12 and 24. In this interim analysis, all patients had completed the week 12 visit; there is incomplete data after week 12.

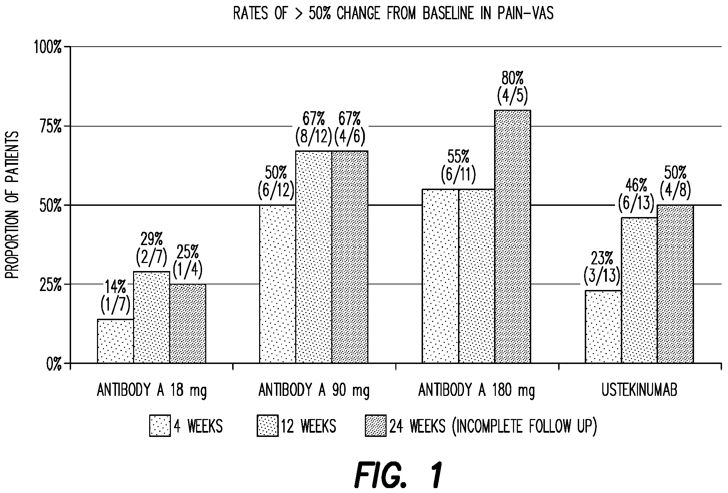

**Results:** The primary endpoint of PASI 90 response at week 12 was achieved by 32.6% (14/43), 73.2% (30/41) and 81.0% (34/42) of Antibody A patients in the 18, 90 and 180 mg groups, respectively, and 40.0% (16/40) of ustekinumab patients. A two-sided Cochran-Mantel-Haenszel test of PASI 90 response at week 12 between the 18, 90 and 180 mg groups of Antibody A and ustekinumab gave p-values of 0.4337, 0.0013 and <0.0001, respectively. Of 166 patients, 46 (27.7%) had concurrent PsA. Median percentage decrease from baseline in pain-VAS at week 12 were 31.9%, 70.2% and 58.4%, respectively, in patients in Antibody A 18, 90 and 180 mg dose groups compared with 57.3% in ustekinumab-treated patients. More than 50% decrease in pain-VAS (defined *post hoc*) at week 12 was achieved in 29% (2/7), 73% (8/11) and 50% (6/12) of patients in Antibody A 18, 90 and 180 mg dose groups, respectively, compared with 54% (7/13) in ustekinumab-treated patients. Figure 1 demonstrates that reductions in pain-VAS score were observed as early as 4 weeks and maintained through 24 weeks (incomplete data). Adverse events (AEs) were similar across treatment groups with no drug-related severe or serious AEs.

**Conclusions:** Selective blockade of IL-23p19 with Antibody A is associated with superior clinical response compared to ustekinumab on skin lesions in patients with moderate-to-severe plaque psoriasis. Numeric improvement over ustekinumab in pain-VAS was also observed in patients with PsA.

PASI 50, PASI 75 and PASI 100 responses at week 12 were also determined in the above study. They showed proportions of patients achieving PASI 50 at week 12 of 93%, 95% and 100% of Antibody A patients in the 18, 90 and 180 mg groups, respectively, and 87% of ustekinumab patients. Achievement of PASI 50 over time is shown in Figure 2.

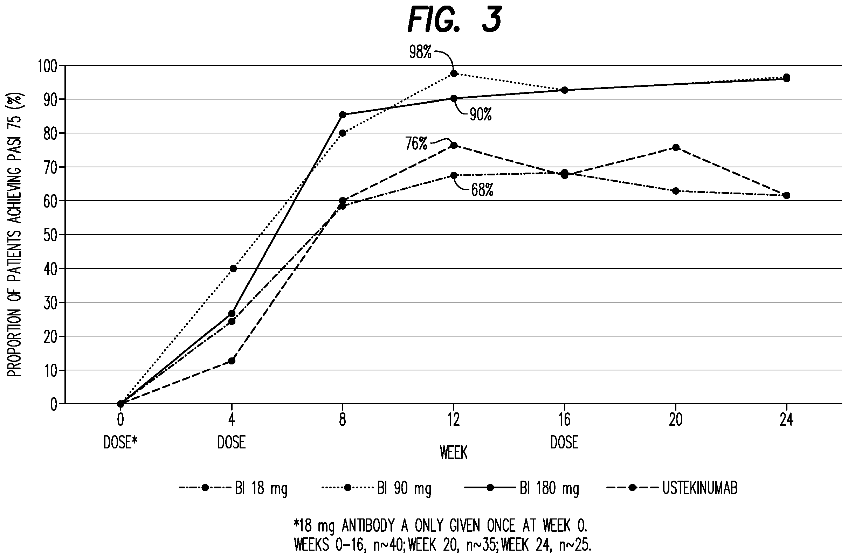

They also showed proportions of patients achieving PASI 75 at week 12 of 76%, 90% and 98% of Antibody A patients in the 18, 90 and 180 mg groups, respectively, and 68% of ustekinumab patients. Achievement of PASI 75 over time is shown in Figure 3.

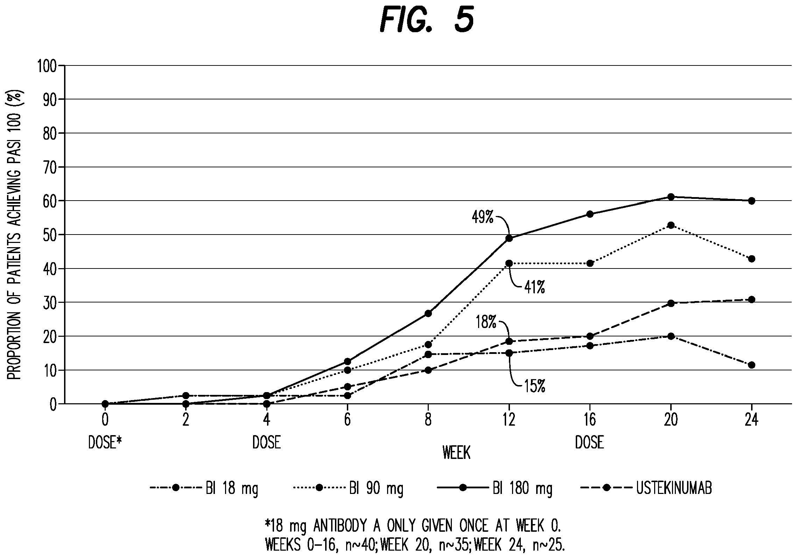

They also showed proportions of patients achieving PASI 100 at week 12 of 18%, 41% and 49% of Antibody A patients in the 18, 90 and 180 mg groups, respectively, and 15% of ustekinumab patients. Achievement of PASI 100 over time is shown in Figure 5.

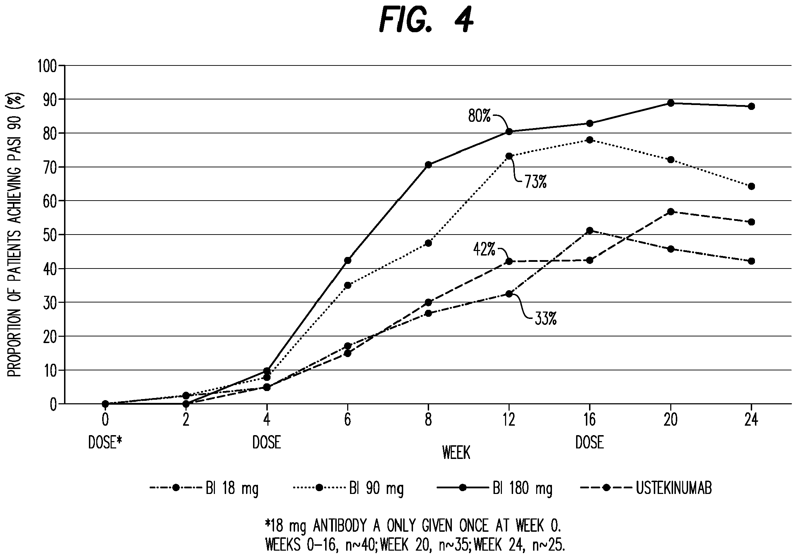

Achievement of PASI 90 over time is shown in Figure 4.

### Example 1c: Study

This study assessed the onset and duration of clinical response following treatment with a selective IL-23p19 inhibitor (Antibody A) compared with ustekinumab in patients with moderate-to-severe chronic plaque psoriasis.

**Materials & Methods:** In this multi-center, randomized, double-blind (within Antibody A dose groups) Phase II study, 166 patients with moderate-to-severe chronic plaque psoriasis were assigned to receive subcutaneous injections of either Antibody A at three different dose regimens (18 mg single injection; 90 or 180 mg at Weeks 0, 4 and 16) or ustekinumab (45 or 90 mg, based on weight, at Weeks 0, 4 and 16). PASI was assessed at Weeks 0, 1, 2, 4, 6 and 8, followed by every 4 weeks for a total of 48 weeks. Safety was assessed at all visits by recording adverse events (AEs).

**Results:** At Week 24, PASI 90 was achieved by 66% (27/41) and 86% (36/42) of patients in the 90 mg and 180 mg Antibody A arms, respectively, compared with 55% (22/40) of patients receiving ustekinumab. Complete clearing of lesions (PASI 100) was achieved in 41%

(17/41) and 57% (24/42) of patients in the 90 and 180 mg Antibody A arms, compared with 28% of patients receiving ustekinumab. The time to onset of PASI 90 was approximately twice as fast for patients in the 90 and 180 mg Antibody A arms compared with those in the ustekinumab arm (median days to onset of PASI 90 = 57 days [in both Antibody A dose groups; ∼8 weeks] vs 113 days [∼16 weeks], respectively, p =0.0016 [90 mg] and p <0.0001 [180 mg]). Patients in the 90 and 180 mg Antibody A arms who achieved PASI 90 maintained that response longer than those receiving ustekinumab (days to 50% of patients having first loss of PASI 90 = 337 days [in both Antibody A dose groups; ∼48 weeks] vs 253 days [∼36 weeks], respectively). AEs were similar between treatment arms and there was no dose response relationship for any AE. Seven patients reported serious AEs (four in the 18 mg and two in the 90 mg Antibody A arms and one in the ustekinumab arm); all were considered unrelated to study medication.

The same study showed that Antibody A had a rapid onset of effect with 69% and 66% of patients (180 mg and 90 mg, respectively) achieving PASI 50 by Week 4, vs 45% of ustekinumab patients. By Week 8, 83% and 80% of Antibody A patients (180 mg and 90 mg, respectively) had achieved PASI 75 vs 60% of ustekinumab patients. By Week 12, the proportion of patients achieving PASI 90 (primary endpoint) was 81% and 73% for 180 mg and 90 mg Antibody A arms vs 40% for ustekinumab. Body weight had little impact on efficacy for Antibody A. At Week 20, 90% and 76% of Antibody A patients (180 mg and 90 mg, respectively) achieved PASI 90 vs 55% for ustekinumab; and 62% and 51% of Antibody A patients had complete clearing of lesions (PASI 100) vs 25% of ustekinumab patients. Clinical responses were sustained at high levels beyond Week 36 for the 180 mg and 90 mg Antibody A dose groups. In patients with PsA, there was a greater decrease in pain at Week 12 observed with Antibody A (180 mg and 90 mg, pooled) vs ustekinumab (68% vs 57%). In patients with psoriatic arthritis, pain was assessed by visual analog scale and expressed as median percentage change from baseline.

**Conclusions:** Selective blockade of IL-23 by Antibody A demonstrates superior efficacy, a more rapid onset and longer duration of response compared with blockade of IL-12 and IL-23 with ustekinumab.

In the same study, Dermatology Life Quality Index (DLQI) and the EuroQol-5D (EQ-5D) were completed at Weeks 0, 12, 24, and 48. The median percentage changes from baseline in DLQI total score (analyzed descriptively and by the van Elteren test) were compared with ustekinumab at Weeks 12 and 24. The proportion of patients achieving a DLQI score of 0 or 1 was also assessed. The EQ-5D index score was analyzed descriptively. Antibody A results are reported for the pooled 90 mg and 180 mg arms.

At baseline, the median DLQI score was 13.0 for patients randomized to Antibody A, compared with 16.0 for those randomized to ustekinumab. Mean EQ-5D index score was 0.7 in both treatment arms. At Week 12, the median percentage improvement in DLQI score was greater for patients receiving Antibody A than for those receiving ustekinumab (100% vs 90.6%, respectively; p=0.0304). The proportion of patients with a DLQI score of 0 or 1 at Week 24 was 80% for Antibody A patients compared with 61% for ustekinumab patients. At Week 12, Antibody A patients were observed to have a 50% greater improvement in mean EQ-5D score compared with ustekinumab patients (0.3 vs 0.2, respectively). This was sustained at Week 24.

In patients with moderate-to-severe plaque psoriasis, selective blockade of IL-23 by Antibody A demonstrated superior efficacy and provides significant improvement in QoL outcomes compared with ustekinumab.

In the same study, the efficacy of Antibody A was assessed for the treatment of scalp, palmoplantar and nail psoriasis.

Psoriasis Scalp Severity Index (PSSI) and Palmoplantar Psoriasis Area and Severity Index (PPASI) were used to evaluate scalp and palmoplantar psoriasis, respectively. Nail Psoriasis Severity Index (NAPSI) was assessed on the hands only. Assessments were performed at baseline and (in those who had symptoms at baseline) at Weeks 1, 2, 4, 6, 8, 12 and every 4 weeks through Week 48. Patients who were documented to have no signs or symptoms were assigned a total score of zero for that visit. Median percentage improvements from baseline for Antibody A (90 mg and 180 mg, pooled) were compared with those for ustekinumab.

One hundred and fifty four (92.8%) patients had scalp psoriasis, 42 (25.3%) patients had palmoplantar psoriasis and 96 (57.8%) patients had nail psoriasis. Scalp and palmoplantar disease responded rapidly; by Week 2, Antibody A-treated patients showed improvements from baseline in PSSI and PPASI of 50% and 51%, compared with 37% and 30% for ustekinumab-treated patients. By Week 6, patients in both Antibody A and ustekinumab groups had complete clearance of scalp psoriasis, which were maintained throughout the study. For PPASI, complete clearance was seen at Week 6 for Antibody A and Week 16 for ustekinumab and maintained throughout the study. Nail disease resolved more slowly, with 30% improvement in NAPSI for Antibody A at Week 6 compared to 0% for ustekinumab. By Week 12, improvements in NAPSI were 41% and 36% for Antibody A and ustekinumab, respectively, and by Week 24, the improvements in NAPSI were 61% and 67%, respectively.

Selective blockade of IL-23 by Antibody A demonstrates promising efficacy in the treatment of scalp, palmoplantar, and nail psoriasis, and suggests a more rapid improvement may be achieved with Antibody A compared with ustekinumab.

### Example 1d: Study

Clinical responses following drug withdrawal and retreatment or switching from ustekinumab to Antibody A are tested.

**Materials & Methods:** Of the 166 patients with moderate-to-severe plaque psoriasis in the above Phase II study, 109 patients who successfully completed 48 weeks, or whose PASI response fell below 50% prior to completing the trial, entered an open-label extension (OLE) study and were treated with Antibody A 90 mg every 12 weeks. Efficacy (PASI 90 and 100) and safety (AEs) were assessed. Non-response imputation was used for missing efficacy data.

**Results:** At the initiation of the OLE, PASI 90 rates were 0% (0/22), 53.6% (15/28), 50.0% (16/32) and 14.8% (4/27) of patients from the Phase II Antibody A (18 mg, 90 mg and 180 mg) and ustekinumab arms, respectively. Twelve weeks after receiving a single injection of 90 mg Antibody A, 59.1% (13/22), 71.4% (20/28), 68.8% (22/32) and 66.7% (18/27) of patients in each arm achieved PASI 90. Among all non-responders (PASI improvement < 50%) from the Phase II study, 48.6% (17/35) achieved PASI 90 response 12 weeks after a single injection of 90 mg Antibody A. In addition, all patients who entered the OLE with a PASI 90 response maintained PASI 90 responses. Similarly, PASI 100 rates at initiation of the OLE were 0% (0/22), 39.3% (11/28), 28.1% (9/32) and 3.7% (1/27) for the 4 Phase II arms, respectively. These increased to 31.8% (7/22), 42.9% (12/28), 40.6% (13/32) and 37.0% (10/27) 12 weeks after a single injection of 90 mg Antibody A. AEs were similar to the preceding Phase II trial. One patient experienced serious AEs (18 mg Antibody A arm) that were considered unrelated to study medication.

**Conclusions:** Patients who had been previously treated with ustekinumab responded well to Antibody A. In addition, re-treatment with Antibody A maintained or improved upon PASI 90 response rates.

### Example 2: Assessment of efficacy

Antibody A is administered to a patient at a dose and dose regimen described herein. The efficacy of Antibody A is assessed by one or more of the following endpoints, which are determined using known methods. Endpoints are measured for example at one or more of week 4, 6, 8, 12, 16, 20, 24, 28, 32, 36, 40, 44, 48, 52, 76 or 104 after administration or as set forth below.

### Psoriasis:

- Achievement of ≥ 90% reduction from baseline PASI score (PASI₉₀), for example at week 12.
- Achievement of ≥75% reduction from baseline in PASI score (PASI₇₅), for example at week 12 and 24.
- Achievement of 100% reduction from baseline in PASI score (PASI₁₀₀), for example at week 12.
- Achievement of ≥ 50% reduction from baseline in PASI score (PASI₅₀), for example at week 12.
- Achievement of PASI₉₀, for example at week 24.
- Achievement of sPGA clear or almost clear, for example at week 12.
- Percentage of PASI reduction from baseline, for example at week 12.
- Time to loss of PASI₅₀ response. This endpoint is calculated from the first treatment to first < 50% reduction of PASI score compared with baseline after the response has been achieved.

Other endpoints are as follows:
- Achievement of PASI₉₀ and PASI₇₅ for example at week 4, 6, 8, 16, 20, 28, 32, 36, 40, 44, 48.
- Achievement of PASI₁₀₀, PASI₅₀ and percentage of PASI reduction from baseline, for example at week 4, 6, 8, 16, 20, 24, 28, 32, 36, 40, 44, 48.
- Achievement of sPGA clear or almost clear, for example at week 4, 6, 8, 16, 20, 24, 28, 32, 36, 40, 44, 48.
- Time to onset of PASI₅₀ response (from the first treatment to first ≥ 50% reduction of PASI score compared with baseline).
- Percent change from baseline in NAPSI (Nail Psoriasis Severity Index), PSSI (Psoriasis Scalp Severity Index) and PPASI (Palmoplantar Psoriasis Area Severity Index), for example at week 4, 6, 8, 12, 16, 20, 24, 28, 32, 36, 40, 44, 48.
- Change from baseline in PGAR (Patient's Global Assessment Rank) and PAI (Patient's Assessment of Itch), for example at week 4, 12, 24, 36, 48.
- Change from baseline in Pain-VAS, for example at week 12 (in subgroup of patients with psoriatic arthritis only).
- Change from baseline in Dermatology Life Quality Index (DLQI), for example at weeks 4, 6, 8, 12, 16, 20, 24, 48.
- Change from baseline in EQ-5D (EuroQoL Group Questionaire) VAS score (Visual Analog Scale), for example at week 12.
- Change from baseline in EQ-5D index score, for example at week 12.
- Proportion of patients achieving Dermatology Life Quality Index (DLQI) scores of 0 or 1, for example at week 12.
- Change from baseline in psoriasis symptoms evaluated using the total score on the PSS (Psoriasis Symptoms Scale), for example at week 16.
- Achievement of total score on the PSS of 0, for example at week 16.

Psoriatic arthritis (responses assessed for example at one or more of week 4, 6, 8, 12, 16, 20, 24, 28, 32, 36, 40, 44, 48, 52, 76, 104):
- Psoriatic Arthritis Screening and Evaluation (PASE) Questionnaire, for example at week 4, 6, 8, 12, 16, 20, 24, 28, 32, 36, 40, 44, 48.
- 20% improvement in American College of Rheumatology response criteria (ACR 20), for example at week 4, 6, 8, 12, 16, 20, 24, 28, 32, 36, 40, 44, 48.
- 50% and 70% improvement in American College of Rheumatology response criteria (ACR 50 and ACR 70).
- Clinical Disease Activity Index (CDAI).
- Disease Activity Score for 28-joint counts (DAS28).
- Dactylitis (swelling of the whole digit), with assessment of the number of all 20 digits that are affected (ranges from 0 to 20 digits (fingers and toes) as present or absent for each digit).
- Enthesitis (inflammation of tendon and ligament insertions, as assessed by the presence or absence of tenderness at the six sites of the Leeds Enthesitis Index (LEI)).
- Individual ACR components including the number of tender or painful joints, the number of swollen joints.
- The patient's global assessment of disease activity and joint pain.
- The physician's global assessment of disease activity.
- Responses of the Health Assessment Questionnaire-Disability Index (HAQ-DI), Health Assessment Questionnaire-Disability Index (HAQ-DI).
- C-reactive protein levels.
- Erythrocyte sedimentation rate.
- Patient-reported outcome measures included the Bath Ankylosing Spondylitis Disease Activity Index (BASDAI). Scores on the Bath Ankylosing Spondylitis Disease Activity Index (BASDAI).
- Version 2 of the Medical Outcomes Study 36-Item Short-Form Health Survey (SF-36).
- Psoriasis Symptom Inventory.
- The modified total Sharp score (mTSS), for example at week 24, 52, 104.
- The tender-joint count.
- The swollen-joint count.
- Patient's assessment of pain,
- Patient's assessment of Level of disability.
- Acute-phase reactant (C-reactive protein or erythrocyte sedimentation rate),
- Patient's global assessment of disease.
- Physician's global assessment of disease.
- Minimal Disease Activity (MDA).
- Composite Psoriatic Disease Activity Index (CPDAI).
- Psoriasis Area and Severity Index (PASI) percentage change from baseline (PASI 75/90/100).
- Modified Nail Psoriasis Severity Index (mNAPSI).
- Psoriatic Arthritis Quality of Life (PsAQOL).
- Functional Assessment of Chronic Illness Therapy-Fatigue (FACIT-F).
- Psoriatic Arthritis Magnetic Resonance Imaging Scoring System (PsAMRIS), for example at week 24, 52 and 104.

For Ankylosing Spondylitis and non-radiographic ax-SpA (responses assessed for example at one or more of week 4, 6, 8, 12, 16, 20, 24, 28, 32, 36, 40, 44, 48, 52, 76, 104):
- Patient-reported inflammatory symptoms.
- Acute-phase reactants (erythrocyte sedimentation rate [ESR] or CRP).
- Bath Ankylosing Spondylitis Disease Activity Index (BASDAI). Change in BASDAI score as compared to baseline.
- Ankylosing Spondylitis Disease Activity Score (ASDAS). Change in ASDAS score compared to baseline.
- Bath Ankylosing Spondylitis Function Index (BASFI). Change in BASFI score as compared to baseline.
- Bath Ankylosing Spondylitis Metrology Index (BASMI). Change in spinal mobility assessed by BASMI score as compared to baseline.
- Magnetic Resonance Imaging (MRI), for example at week 24, 52, 104.
- ASAS 40 response.
- ASAS 5/6 response.
- ASAS partial remission criteria.
- ASAS 20 response
- Change in peripheral joint count (TJC and SJC) as compared to baseline.
- Change in entheseal score (MASES) at as compared to baseline.
- Change in Ankylosing Spondylitis Disease Activity Score (ASDAS) score as compared to baseline.
- Time to loss of maintenance.
- Time to post-treatment flare.
- Time to loss of maintenance.
- Ankylosing Spondylitis Quality of Life scale (ASQoL).

### Example 3: Pharmacokinetic endpoint(s)

In order to determine the pharmacokinetic parameters of an anti-IL-23A antibody, such as Antibody A, the following parameters are evaluated using methods known in the art:
- maximum measured plasma concentration (Cmax)
- time from dosing to the maximum plasma concentration (tmax)
- area under the plasma concentration-time curve over the time interval of treatment (AUC0-t)
- area under the plasma concentration-time curve over the time interval from zero extrapolated to infinity (AUC0-∞)
- terminal half-life (t1/2)
- population pharmacokinetics.

### Example 4: Pharmaceutical compositions

Examples of formulations suitable for an antibody of the present invention are shown below. Antibodies used in the formulations below are for example Antibody A, Antibody B, Antibody C or Antibody D.

**Formulation 1:**

| **Components** | **Concentration [mmol/L]** | **Concentration [g/l]** | **Nominal Amount [mg/vial]** |
|---|---|---|---|
| | | | **V = 10.0 ml** |
| Antibody | | 10.0 | 100.0 |
| Succinic acid | 0.7 | 0.083 | 0.8 |
| Disodium succinate hexahydrate | 24.3 | 6.564 | 65.6 |
| Sodium chloride | 125 | 7.305 | 73.1 |
| Polysorbat 20 | 0.16 | 0.20 | 0.20 |
| Water for Injection | - | Ad 1L | Ad 1mL |

The pH of formulation 1 is typically in the range of pH 6.0 to 7.0, for example pH 6.5. This formulation is particularly suitable for intravenous administration.

Molecular weight (MW in g/mol) of used excipients: Disodium succinate hexahydrate = 270.14 g/mol; Succinic acid = 118.09 g/mol; Sodium chloride = 58.44 g/mol.

The osmolarity of the formulation is 300 +/- 30 mOsmol/kg, as determined using an Osmomat 030 (Gonotec GmbH, Berlin, Germany). The density at 20°C of the formulation is approximately 1.0089 g/cm³, as determined using a measuring unit DMA 4500 (Anton Paar GmbH, Ostfildern-Scharnhausen, Germany).

**Formulation 2:**

| **Components** | **Concentration [mmol/L]** | **Concentration [g/l]** | **Nominal Amount [mg/syringe]** |
|---|---|---|---|
| | | | **V = 1.0 ml** |
| Antibody | 0.6 | 90.0 | 90.0 |
| Succinic acid | 0.5 | 0.059 | 0.059 |
| Disodium succinate hexahydrate | 3.9 | 1.054 | 1.054 |
| Sorbitol | 225 | 41.00 | 41.00 |
| Polysorbat 20 | 0.16 | 0.20 | 0.20 |
| Water for Injection | - | Ad 1L | Ad 1mL |

The pH of formulation 2 is typically in the range of pH 5.5 to 6.5, for example 5.5 to 6.1, for example the pH is 5.8. This formulation is particularly suitable for subcutaneous administration.

Molecular weight (MW in g/mol) of used excipients:
MW: Succinic acid (C₄H₆O₄)= 118.09 g/mol
MW: Disodium succinate hexahydrate (C₄O₄Na₂H₄ x 6H₂O) = 270.14 g/mol
MW: Sorbitol = 182.17 g/mol
MW: Polysorbate 20 = 1227.72 g/mol

The osmolarity of the formulation is 300 +/- 30 mOsmol/kg, as determined using an Osmomat 030 (Gonotec GmbH, Berlin, Germany). The density at 20°C of the formulation is approximately 1.040 g/cm³, as determined using a measuring unit DMA 4500 (Anton Paar GmbH, Ostfildern-Scharnhausen, Germany).

**Formulation 3:**

| **Components** | **Concentration [mmol/L]** | **Concentration [g/l]** | **Nominal Amount [mg/syringe]** |
|---|---|---|---|
| | | | **V = 1.0 ml** |
| Antibody | 0.6 | 90.0 | 90.0 |
| Sorbitol | 240 | 43.733 | 43.733 |
| Polysorbat 20 | 0.16 | 0.20 | 0.20 |
| Water for Injection | - | Ad 1L | Ad 1mL |

The pH of formulation 3 is typically in the range of pH 5.5 to 6.5, for example 5.5 to 6.1, for example the pH is 5.8. This formulation is particularly suitable for subcutaneous administration.

Molecular weight (MW in g/mol) of used excipients:
MW: Sorbitol = 182.17 g/mol
MW: Polysorbate 20 = 1227.72 g/mol.

The osmolarity of the formulation is 300 +/- 30 mOsmol/kg, as determined using an Osmomat 030 (Gonotec GmbH, Berlin, Germany).

### Example 5: Molecular and histopathological profile

The molecular and histopathological profile in skin lesions from psoriasis patients treated with Antibody A vs ustekinumab were compared.

**Materials & Methods:** Lesional skin biopsies were obtained in two clinical trials of moderate-to-severe psoriasis patients, a Ph I study of single doses of Antibody A (0.01 mg/kg to 5 mg/kg) vs placebo and the Ph II study described above in Examples 1a to 1c comparing treatment with Antibody A (18 mg single injection; 90 mg or 180 mg at Wks 0, 4 and 16) vs ustekinumab (45 mg or 90 mg at Wks 0, 4 and 16). Samples were collected from 39 patients in the Ph I study (at baseline and Wk 8) and 60 patients in the Ph II study (at baseline and Wk 4) for histopathology and transcriptome-wide RNA profiling. Univariate associations were assessed using linear regression. Effect size, p-values and FDR were calculated for significant transcripts.

**Results:** As early as Wk 4, Antibody A treatment decreased expression of histopathology biomarkers including K16, Ki67, CD3, neutrophil gelatinase lipocalin, CD11c, DC-LAMP, b-defensin 2 and S100A7. In the Ph II study, 50% (7/14) and 64% (9/14) of patients on 90 or 180 mg Antibody A, respectively, were graded as excellent improvement (based on a global histopathology score) compared with 27% (4/15) for ustekinumab. Expression of a selected set of genes was significantly decreased in lesions at Wk 4 in those patient samples that were graded as excellent improvement vs other categories. The pathways altered included type I IFN, S-100, keratinocyte and late cornified envelope family. Treatment with Antibody A resulted in significant decreases (p<0.005) from baseline to Wk 4 in the expression of 1714 unique genes, including IL-23R, CCL20, EL-36β, β-defensin 4B, and type I IFN pathway and late cornified envelope family genes, in patients who received Antibody A but not ustekinumab. Expression of a set of 1709 genes was significantly decreased by both treatments. At 4 and 8 wks, Antibody A strongly downregulated expression of genes associated with keratinocytes and epidermal cells and, to a lesser extent, those associated with monocytes, macrophages, dendritic cells and neutrophils. Table 7 shows selected genes decreased by Antibody A (Bonferroni correction was applied for multiple comparisons).

**Conclusions:** The superior clinical efficacy of Antibody A was associated with early changes in the molecular and histopathological profile of skin lesions in psoriasis patients compared with ustekinumab.

Skin biopsies were collected from 58 patients at baseline and at Week 4. Biomarkers associated with psoriatic disease and the IL-23 pathway were assessed in skin biopsies, pre- and post-treatment, by immunohistochemistry. Biopsy results were classified as excellent, good, slight, no change, or worsening (see Table 6A for classification criteria). A summary of global histopathologic scores by treatment group in a subset of patients who had lesional biopsies collected at baseline and at Week 4 is shown in Table 6B. The scores are based on the classifications in Table 6A. Only 52 biopsies were assessed, because some of the biopsies were either not assessable or missing at baseline or Week 4 and thus excluded from the analysis.

Additionally, transcriptome-wide RNA sequencing was performed on biopsy samples.

Global transcriptome-wide sequencing of RNA from lesion skin biopsy samples from a subset of 58 patients was achieved using the Illumina Hi-Seq 2000 (Illumina Inc., San Diego, CA). A generalized linear model of the RNA seq data for each gene was fitted using empirical Bayes estimates of the dispersion (variance). The model included the change in log gene expression as the dependent variable and treatment group as the independent variable. This model yielded estimates of the adjusted log fold-change associated with treatment and an associated p-value for each RNA transcript. P-values of <0.005 were considered significant. The experiment-wise false discovery rate was computed according to standard methods using Bonferroni correction applied for multiple comparisons.

**Table 6A. Immunohistochemistry Grading Classification Criteria**

| **Grading** | **Classification Criteria** |
|---|---|
| Excellent improvement | Disease reversal or complete improvement by histological criteria. Epidermal acanthosis is reversed, terminal differentiation abnormalities (lack of granular layer, parakeratosis) are reversed, keratin 16 is no longer expressed by supra basal keratinocytes (thus regenerative epidermal hyperplasia is eliminated), and tissue patterning no longer has psoriasis-like features. Histologically, the treated lesion looks like non-lesional skin. If examined by histopathology, psoriasis would not be diagnosed as an underlying disease. |
| Good improvement | Strong attenuation of psoriasis features, but there is focal keratin 16 expression, indicating that some regions of the epidermis maintain the regenerative epidermal hyperplasia phenotype. Epidermal acanthosis will be reduced, epidermal differentiation improved, and the overall biopsy will have weak psoriasis-like features. If examined by histopathology, a clear diagnosis of psoriasis would not be given. The biopsy will probably not look like non-lesional skin, but will retain focal features of the psoriatic process. |
| Slight improvement | Partial attenuation of psoriasis features. Epidermis will be thinner than the pre-treatment biopsy and some improvement in epidermal differentiation will be evident, but the biopsy will retain uniform keratin 16 staining in supra basal keratinocytes, so the epidermis is displaying regenerative epidermal hyperplasia. |
| No change | No histological change from pre-treatment biopsy. |
| Worsening | Increased acanthosis of the epidermis (more epidermal hyperplasia than baseline). |

**Table 6B. Summary of global histopathologic scores**

| **N (%)** | **Antibody A** | | | **Ustekinumab 45/90 mg (N=14)** |
|---|---|---|---|---|
| | **18 mg (N=12)** | **90 mg (N=13)** | **180 mg (N=13)** | |
| Excellent improvement | 3 (25) | 7 (54) | 9 (69) | 4 (29) |
| Good improvement | 4 (33) | 2 (15) | 3 (23) | 2 (14) |
| Slight improvement | 4 (33) | 1 (8) | 1 (8) | 6 (43) |
| No change | 1 (8) | 3 (23) | 0 | 1 (7) |
| Worsening | 0 | 0 | 0 | 1 (7) |

Table 6C shows the numbers of patients in PASI response over time with a mean decrease in each of 3423 significant genes at Week 4, the expression of which was significantly decreased after treatment with Antibody A (1714 genes) and after treatment with Antibody A and treatment with ustekinumab (1709 genes).

Further analysis showed that the expression of 440 genes was significantly decreased by Antibody A at week 4 with a > 2 fold change (baseline to week 12). Only 89 of these genes showed decreased expression with treatment with Antibody A, while reductions in the expression of 351 overlapping genes between treatment Antibody A and treatment with ustekinumab was observed. The 89 genes are listed in Table 6D.

**Table 6C.**

| **Numbers of patients in PASI response over time with a mean decrease in each of 3423 significant genes at Week 4** | | | |
|---|---|---|---|
| PASI response | Week 16 | Week 24 | Week 48 |
| PASI 75 | 39/41 (95.1%) | 38/41 (92.7%) | 29/41 (70.7%) |
| PASI 90 | 35/41 (85.4%) | 31/41 (75.6%) | 20/41 (48.8%) |
| PASI 100 | 22/41 (53.7%) | 22/41 (53.7%) | 11/41 (26.8%) |

The key pathways modulated by Antidody A versus ustekinumab at week 4 are as follows (p-value<0.005 & fold change >1.5 for the genes included in these pathways):

Stronger enrichment/modulation for Antibody A:
Hypoxia, myogenesis, UV response DN, angiogenesis, coagulation, xenobiotic metabolism, estrogen response late, G2M checkpoint, estrogen response early, epithelial mesenchymal transition.

Stronger enrichment/modulation for ustekinumab:
IL2 STAT5 signaling, Interferon_Alpha_response, Interferon Gamma response, IL6 JAK STAT3 signaling, inflammatory response, TNFA signaling via NFKB.

**Table 6D.**

| | |
|---|---|
| NPC1L1 | NPC1-like 1 [Source:HGNC Symbol;Acc:7898] |
| ARSF | arylsulfatase F [Source:HGNC Symbol;Acc:721] |
| CHI3L2 | chitinase 3-like 2 [Source:HGNC Symbol;Acc: 1933] |
| PITX1 | paired-like homeodomain 1 [Source:HGNC Symbol;Acc:9004] |
| CNGB1 | cyclic nucleotide gated channel beta 1 [Source:HGNC Symbol; Acc:2151] |
| SLC12A3 | solute carrier family 12 (sodium/chloride transporters), member 3 [Source:HGNC Symbol;Acc: 10912] |
| ATP12A | ATPase, H+/K+ transporting, nongastric, alpha polypeptide [Source:HGNC Symbol;Acc:13816] |
| ARG2 | arginase, type II [Source:HGNC Symbol;Acc:664] |
| VNN3 | vanin 3 [Source:HGNC Symbol;Acc: 16431] |
| SERPIND1 | serpin peptidase inhibitor, clade D (heparin cofactor), member 1 [Source:HGNC Symbol;Acc:4838] |
| OLFM4 | olfactomedin 4 [Source:HGNC Symbol;Acc: 17190] |
| DKK4 | dickkopf homolog 4 (Xenopus laevis) [Source:HGNC Symbol; Acc:2894] |
| SIGLEC5 | sialic acid binding Ig-like lectin 5 [Source:HGNC Symbol; Acc:10874] |
| HYAL4 | hyaluronoglucosaminidase 4 [Source:HGNC Symbol;Acc:5323] |
| GNAT1 | guanine nucleotide binding protein (G protein), alpha transducing activity polypeptide 1 [Source:HGNC Symbol;Acc:4393] |
| CCL20 | chemokine (C-C motif) ligand 20 [Source:HGNC Symbol;Acc: 10619] |
| C11orf9 | chromosome 11 open reading frame 9 [Source:HGNC Symbol; Acc:1181] |
| SRMS | src-related kinase lacking C-terminal regulatory tyrosine and N-terminal myristylation sites [Source:HGNC Symbol;Acc: 11298] |
| TGM3 | transglutaminase 3 (E polypeptide, protein-glutamine-gamma-glutamyltransferase) [Source:HGNC Symbol;Acc: 11779] |
| TEX101 | testis expressed 101 [Source:HGNC Symbol;Acc:30722] |
| ACRV1 | acrosomal vesicle protein 1 [Source:HGNC Symbol;Acc:127] |
| EHF | ets homologous factor [Source:HGNC Symbol;Acc:3246] |
| NKX2-8 | NK2 homeobox 8 [Source:HGNC Symbol;Acc: 16364] |
| REN | renin [Source:HGNC Symbol;Acc:9958] |
| ANKRD31 | ankyrin repeat domain 31 [Source:HGNC Symbol;Acc:26853] |
| GBAP1 | glucosidase, beta, acid pseudogene 1 [Source:HGNC Symbol; Acc:4178] |
| PGLYRP2 | peptidoglycan recognition protein 2 [Source:HGNC Symbol; Acc:30013] |
| IL23R | interleukin 23 receptor [Source:HGNC Symbol;Acc: 19100] |
| LCE3D | late cornified envelope 3D [Source:HGNC Symbol;Acc:16615] |
| PPBP | pro-platelet basic protein (chemokine (C-X-C motif) ligand 7) [Source:HGNC Symbol;Acc:9240] |
| BMPER | BMP binding endothelial regulator [Source:HGNC Symbol; Acc:24154] |
| CLEC3A | C-type lectin domain family 3, member A [Source:HGNC Symbol; Acc:2052] |
| A2ML1 | alpha-2-macroglobulin-like 1 [Source:HGNC Symbol;Acc:23336] |
| TGM6 | transglutaminase 6 [Source:HGNC Symbol;Acc:16255] |
| MTNR1A | melatonin receptor 1A [Source:HGNC Symbol;Acc:7463] |
| MZB1 | marginal zone B and B1 cell-specific protein [Source:HGNC Symbol;Acc:30125] |
| CEACAM3 | carcinoembryonic antigen-related cell adhesion molecule 3 [Source:HGNC Symbol;Acc:1815] |
| REG3A | regenerating islet-derived 3 alpha [Source:HGNC Symbol; Acc:8601] |
| ABCG4 | ATP-binding cassette, sub-family G (WHITE), member 4 [Source: HGNC Symbol;Acc:13884] |
| SLC22A13 | solute carrier family 22 (organic anion transporter), member 13 [Source:HGNC Symbol;Acc:8494] |
| MYO1H | myosin IH [Source:HGNC Symbol;Acc: 13879] |
| GCNT4 | glucosaminyl (N-acetyl) transferase 4, core 2 [Source:HGNC Symbol;Acc:17973] |
| HMGN2P4 6 | high mobility group nucleosomal binding domain 2 pseudogene 46 [Source:HGNC Symbol;Acc:26817] |
| ALOX12B | arachidonate 12-lipoxygenase, 12R type [Source:HGNC Symbol; Acc:430] |
| MRGPRX3 | MAS-related GPR, member X3 [Source:HGNC Symbol;Acc: 17980] |
| FAM181B | family with sequence similarity 181, member B [Source:HGNC Symbol;Acc:28512] |
| MAFA | v-maf musculoaponeurotic fibrosarcoma oncogene homolog A (avian) [Source:HGNC Symbol;Acc:23145] |
| TREX2 | three prime repair exonuclease 2 [Source:HGNC Symbol; Acc:12270] |
| ADAP2 | ArfGAP with dual PH domains 2 [Source:HGNC Symbol; Acc:16487] |
| KRT76 | keratin 76 [Source:HGNC Symbol;Acc:24430] |
| LCE3E | late cornified envelope 3E [Source:HGNC Symbol;Acc:29463] |
| CYP4F2 | cytochrome P450, family 4, subfamily F, polypeptide 2 [Source: HGNC Symbol;Acc:2645] |
| KLK12 | kallikrein-related peptidase 12 [Source:HGNC Symbol;Acc:6360] |
| TMPRSS11 A | transmembrane protease, serine 11A [Source:HGNC Symbol; Acc:27954] |
| CCK | cholecystokinin [Source:HGNC Symbol;Acc:1569] |
| ERC2 | ELKS/RAB6-interacting/CAST family member 2 [Source:HGNC Symbol;Acc:31922] |
| RNF222 | ring finger protein 222 [Source:HGNC Symbol;Acc:34517] |
| ZFP92 | zinc finger protein 92 homolog (mouse) [Source:HGNC Symbol; Acc:12865] |
| HIST1H3C | histone cluster 1, H3c [Source:HGNC Symbol;Acc:4768] |
| HIST1H2B H | histone cluster 1, H2bh [Source:HGNC Symbol;Acc:4755] |
| FCHSD1 | FCH and double SH3 domains 1 [Source:HGNC Symbol; Acc:25463] |
| MUC2 | mucin 2, oligomeric mucus/gel-forming [Source:HGNC Symbol; Acc:7512] |
| TRIM40 | tripartite motif containing 40 [Source:HGNC Symbol;Acc: 18736] |
| PTCHD2 | patched domain containing 2 [Source:HGNC Symbol;Acc:29251] |
| CTXN3 | cortexin 3 [Source:HGNC Symbol;Acc:31110] |
| MFSD2B | major facilitator superfamily domain containing 2B [Source:HGNC Symbol;Acc:37207] |
| C22orf42 | chromosome 22 open reading frame 42 [Source:HGNC Symbol; Acc:27160] |
| TRAV8-4 | T cell receptor alpha variable 8-4 [Source:HGNC Symbol; Acc:12149] |
| IGHE | immunoglobulin heavy constant epsilon [Source:HGNC Symbol; Acc:5522] |
| IGHV1-3 | immunoglobulin heavy variable 1-3 [Source:HGNC Symbol; Acc:5552] |
| KLK9 | kallikrein-related peptidase 9 [Source:HGNC Symbol;Acc:6370] |
| CPLX3 | complexin 3 [Source:HGNC Symbol;Acc:27652] |
| C3orf43 | chromosome 3 open reading frame 43 [Source:HGNC Symbol; Acc:27407] |
| AZGP1P2 | alpha-2-glycoprotein 1, zinc-binding pseudogene 2 [Source:HGNC Symbol;Acc:912] |
| PLIN5 | perilipin 5 [Source:HGNC Symbol;Acc:33196] |
| SMARCE1 P6 | SWI/SNF related, matrix associated, actin dependent regulator of chromatin, subfamily e, member 1 pseudogene 6 [Source:HGNC Symbol;Acc:39737] |
| ZNF812 | zinc finger protein 812 [Source:HGNC Symbol;Acc:33242] |
| RPL26P34 | ribosomal protein L26 pseudogene 34 [Source:HGNC Symbol; Acc:36249] |
| CYP4A22-AS1 | CYP4A22 antisense RNA 1 [Source:HGNC Symbol;Acc:43715] |
| HK2P1 | hexokinase 2 pseudogene 1 [Source:HGNC Symbol;Acc:4924] |
| LINC00659 | long intergenic non-protein coding RNA 659 [Source:HGNC Symbol;Acc:44316] |
| SERPINH1 P1 | serpin peptidase inhibitor, clade HI, pseudogene 1 [Source:HGNC Symbol;Acc:19917] |
| CSF2RBP1 | colony stimulating factor 2 receptor, beta, low-affinity (granulocyte-macrophage) pseudogene 1 [Source:HGNC Symbol;Acc:2437] |
| OR5H8P | olfactory receptor, family 5, subfamily H, member 8 pseudogene [Source:HGNC Symbol;Acc: 14773] |
| C8orf56 | chromosome 8 open reading frame 56 [Source:HGNC Symbol; Acc:28595] |
| FABP5P11 | fatty acid binding protein 5 pseudogene 11 [Source:HGNC Symbol; Acc:19328] |
| UGT1A8 | UDP glucuronosyltransferase 1 family, polypeptide A8 [Source: HGNC Symbol;Acc: 12540] |
| AOX2P | aldehyde oxidase 2 pseudogene [Source:HGNC Symbol; Acc:18450] |
| LCE3C | late cornified envelope 3C [Source:HGNC Symbol;Acc: 16612] |

**Table 7. Select Genes Decreased by Antibody A at 4 Weeks Post Treatment as Assessed by RNAseq**

| **Differentially expressed gene by RNAseq** | **Protein name** | **Antibody A treated (pooled 90 and 180 mg)** | |
|---|---|---|---|
| | | **Mean Log₂ fold change (vs. baseline)** | **Adjusted P-value** |
| **IL-23/IL-17 pathway related** | | | |
| *IL-23R* | Interleukin 23 receptor | -0.05 | 0.001 |

| **Epidermal hyperplasia** | | | |
|---|---|---|---|
| *IL-36B* | Interleukin 36 β | -0.26 | 0.003 |
| *DEFB4B* | β-defensin 4B | -1.89 | 1.02x10⁻⁷ |

| **Epithelial cell differentiation** | | | |
|---|---|---|---|
| | Late cornified envelope proteins | | |
| *LCE3A* | 3A | -2.7 | 1.85x10⁻¹⁰ |
| *LCE3B* | 3B | -0.96 | 1.65x10⁻⁵ |
| *LCE3C* | 3C | -1.84 | 3.85x10⁻⁶ |
| *LCE3D* | 3D | -1.53 | 7.08x10⁻¹⁰ |
| *LCE3E* | 3E | -1.68 | 1.04x10⁻¹⁰ |

| **Immune-related pathways** | | | |
|---|---|---|---|
| *IFI27* | IFN α-inducible protein 27 | -0.87 | 1.53x10⁻⁸ |
| *IFI27L2* | IFN α-inducible protein 27-like protein 2 | -0.12 | 0.007 |
| *IFI44L* | IFN-induced protein44-like | -1.01 | 6.63x10⁻⁶ |
| *IFIT1* | IFN-induced protein with tetratricopeptide repeats 1 | -0.90 | 2.38X10⁻⁶ |
| *IRF9* | IFN regulatory factor 9 | -0.22 | 8.0x10⁻⁵ |

### Example 6: Immunogenicity Assessments

Immunogenicity of Antibody A was assessed using a multi-tiered approach. All samples were first analyzed in the anti-drug antibody (ADA) screening assay. Samples found to be putative positive in the ADA screening assay were then assessed in the ADA confirmatory assay. Samples that were confirmed positive were then titrated and further characterized in the neutralizing anti-drug antibody (Nab) screening assay. Samples that were found to be putative positive in the Nab screening assay were then evaluated in the Nab specificity assay.

The ADA screening assay began with acid dissociation of the sample, followed by a validated drug-bridging electrochemiluminescence (ECL) assay. Samples and controls were treated with 300 mM acetic acid to dissociate the Antibody A ADA complex. The acid-treated samples were then incubated and neutralized with 1 M Tris pH 9.5 in a mixture of biotinylated-Antibody A and ruthenium-Antibody A. The neutralized samples were then added to the blocked Meso Scale Discovery® (MSD) streptavidin plate and incubated. After washing, the drug-ADA complex was detected by the addition of MSD read buffer to the plate and subsequent excitation of the ruthenium via an electrochemical reaction to generate Ru(bpy)₃²⁺ luminescence that was read by the MSD Sector Imager 6000. The ECL signal intensity correlated with the level of anti-Antibody A antibodies present in the sample. A monoclonal antibody raised against Antibody A was used as the positive control (PC). The sensitivity assessed during validation was 0.177 ng/mL in 100% human K₃EDTA plasma. The drug tolerance was such that 100 ng/mL of PC in 100% human K₃EDTA plasma could be detected in the presence of 100 µg/mL or less of Antibody A.

The validated Nab assay measured neutralizing antibodies to Antibody A in human plasma through assessment of STAT3 phosphorylation. Plasma samples were incubated with a fixed concentration of Antibody A and IL-23. The human large B cell lymphoma cell line, DB, used in the assay displayed increased phosphorylated STAT3 in the presence of IL-23. Phosphorylated-STAT3 was evaluated using the PathScan® Phospho-Stat3 (Tyr705) sandwich ELISA that was read using a SpectraMax Plus³⁸⁴ Absorbance Microplate Reader. In the absence of neutralizing antibodies, Antibody A bound to IL-23 resulted in decreased IL-23R activation and reduced STAT3 phosphorylation. In the presence of neutralizing antibodies, increased IL-23-mediated IL-23R activation and consequent STAT3 phosphorylation occurred.

A NAb specificity assay was also used in order to confirm that a reduction in signal observed in the NAb screening assay was due to antibodies and not due to non-specific matrix components. A monoclonal antibody raised against Antibody A was used as the PC. The relative assay sensitivity assessed during validation was 125 ng/mL in 100% human K₃EDTA plasma. The drug tolerance was such that 250 ng/mL of PC in 100% human K₃EDTA plasma could be detected in the presence of 70 ng/mL or less of Antibody A.

## Claims

1. A method for treating psoriasis, said method comprising administering Antibody A to a patient previously treated with a first agent, wherein the PASI score or sPGA score of said patient improves after the administration of Antibody A.

2. The method of claim 1, wherein said patient reaches PASI 90 after the administration of Antibody A.

3. The method of claim 1, wherein said patient reaches PASI 100 after the administration of Antibody A.

4. The method of claim 1, wherein said patient reaches a sPGA score of clear (score of 0) or almost clear (score of 1) after the administration of Antibody A.

5. The method of claim 1, wherein said first agent is a biological drug, for example a monoclonal antibody.

6. The method of claim 1, wherein said first agent is ustekinumab or adalimumab.

7. A method for treating psoriasis, said method comprising:
a) identifying a patient treated with a first agent, wherein the therapeutic effect of said first agent in said patient is insufficient to treat psoriasis; and
b) administering Antibody A to said patient, wherein the therapeutic effect of Antibody A in said patient is sufficient to treat psoriasis.

8. The method according to claim 7, wherein said patient does not reach PASI 90 before the administration of Antibody A.

9. The method according to claim 7, wherein said patient does not reach PASI 75 before the administration of Antibody A.

10. The method according to claim 7, wherein said patient does not reach PASI 50 before the administration of Antibody A.

11. The method of claim 7, wherein said patient does not reach a sPGA score of clear (score of 0) or almost clear (score of 1) before the administration of Antibody A

12. The method of claim 7, wherein said patient reaches PASI 90 after the administration of Antibody A.

13. The method of claim 7, wherein said patient reaches PASI 100 after the administration of Antibody A.

14. The method of claim 7, wherein said patient reaches a sPGA score of clear (score of 0) or almost clear (score of 1) after the administration of Antibody A.

15. The method of claim 7, wherein said first agent is a biological drug, for example a monoclonal antibody.

16. The method of claim 7, wherein said first agent is ustekinumab or adalimumab.

17. A method for treating psoriasis comprising switching a patient who does not fully respond to a first agent from treatment with said first agent to treatment with Antibody A.

18. The method according to claim 17, wherein a patient does not fully respond when a PASI score of 90 is not reached.

19. The method according to claim 17, wherein a patient does not fully respond when a PASI score of 75 is not reached.

20. The method according to claim 17, wherein a patient does not fully respond when a PASI score of 50 is not reached.

21. The method of claim 17, wherein a patient does not fully respond when a sPGA score of clear (score of 0) or almost clear (score of 1) is not reached..

22. The method of claim 17, wherein said first agent is a biological drug, for example a monoclonal antibody.

23. The method of claim 17, wherein said first agent is ustekinumab or adalimumab.

24. A method for treating generalized pustular psoriasis (GPP) comprising administering to a patient an anti-IL-23A antibody.

25. The method according to claim 24, wherein said anti-IL-23A antibody is Antibody A.

26. A method for detecting the presence or absence of a beneficial response in a patient after administration of an IL-23A antagonist comprising:
a) obtaining a biological sample from the patient;
b) measuring in said sample the level of expression of one or more genes;
c) comparing the level in b) to the level of expression of the one or more genes in a control; and
d) determining whether or not the difference in levels between the sample and the control reflects a beneficial response in the patient, wherein the one or more genes is IL-23R, CCL20, IL-36β, β-defensin 4B, type I IFN pathway genes or late cornified envelope family genes.

27. The method according to claim 26, wherein the patient suffers from psoriasis.

28. The method according to claim 26, the control is from one or more subjects treated with another agent.

29. The method according to claim 26, wherein the IL-23A antagonist is an anti-IL-23A antibody or an antigen binding fragment thereof.

30. The method according to claim 26, wherein the IL-23A antagonist is Antibody A.

31. A method for treating psoriasis or psoriatic arthritis comprising administering to a patient an anti-IL-23A antibody, said method comprising:
a) administering an initial dose of said anti-IL-23A antibody to the patient; and
b) administering a second dose of said anti-IL-23A antibody to the patient when the patient no longer maintains a sPGA score of clear (score of 0) or almost clear (score of 1).

32. The method according to claim 31, wherein the IL-23A antibody is Antibody A.

33. A method for treating psoriasis or psoriatic arthritis comprising administering to a patient an anti-IL-23A antibody, said method comprising:
a) administering an initial dose of said anti-IL-23A antibody to the patient;
b) administering a first maintenance dose of said anti-IL-23A antibody up to 32 weeks after said initial dose is administered; and
c) administering at least one additional maintenance dose to the patient up to 32 weeks after said first maintenance dose is administered.

34. The method according to claim 33, wherein said first maintenance dose is administered 28 weeks or 32 weeks after said initial dose is administered and said at least one additional maintenance dose is administered to the patient 28 weeks or 32 weeks after said first maintenance dose is administered.

35. The method according to claim 33, wherein the IL-23A antibody is Antibody A.

36. A method for treating psoriasis or psoriatic arthritis comprising administering to a patient an anti-IL-23A antibody, said method comprising:
a) administering an initial dose of said anti-IL-23A antibody to the patient;
b) administering a loading dose of said anti-IL-23A antibody to the patient 1 to 6 weeks after said initial dose is administered;
c) administering a first maintenance dose of said anti-IL-23A antibody to the patient up to 32 weeks after said loading dose is administered; and
d) administering at least one additional maintenance dose to the patient up to 32 weeks after said first maintenance dose is administered.

37. The method according to claim 36, wherein said first maintenance dose is administered 28 weeks or 32 weeks after said loading dose is administered and said at least one additional maintenance dose is administered to the patient 28 weeks or 32 weeks after said first maintenance dose is administered.

38. The method according to claim 36, wherein the IL-23A antibody is Antibody A.
